# EUROPEAN PATENT SPECIFICATION

(11) **EP 1 263 430 B1**
(45) Date of publication and mention of the grant of the patent: **05.10.2005**
(21) Application number: 01911855.3
(22) Date of filing: 06.03.2001
(51) Int. Cl.: A61K 31/275, A61K 39/395, A61K 31/7088, A61K 48/00, G01N 33/50, A61P 31/00, A61P 29/00, A61P 19/02, A61P 1/00

(54) **TREATMENT OF DISEASES ASSOCIATED WITH CYTOKINE PRODUCTION WITH INHIBITORS OF THE TEC FAMILY OF PROTEIN TYROSINE KINASES**
BEHANDLUNG CYTOKIN-VERMITTELTER KRANKHEITEN MIT TEC FAMILIE PROTEIN TYROSIN KINASE INHIBITOREN
TRAITEMENT DES MALADIES LIEES A LA PRODUCTION DE CYTOKINES PAR DES INHIBITEURS DES PROTEINES TYROSINE KINASES DE LA FAMILLE TEC

(30) Priority: 06.03.2000 GB 0005345
(43) Date of publication of application: 11.12.2002
(73) Proprietor: Synovis Limited, London EC4A 1JP (GB)
(72) Inventor: FOXWELL, Brian M.J., Mathilda and T. Kennedy Inst., London W6 8LH (GB)
(74) Representative: Campbell, Patrick John Henry
(86) International application number: PCT/GB2001/000949
(87) International publication number: WO 2001/066107

(56) References cited:
- WO-A-00/56737
- WO-A-99/54286
- KAWAKAMI, T. ET AL.: "An inhibitor of Bruton's tyrosine kinase (BTK) from a fungal extract" FASEB J., vol. 13, no. 4, part 1, 12 March 1999 (1999-03-12), page A324 XP002182794
- TIBBLES H E ET AL: "INHIBITION OF COLLAGEN-INDUCED PLATELET AGGREGATION BY SELECTIVELY TARGETING BRUTON'S TYROSINE KINASE WITH LFM-A13" BLOOD, W.B.SAUNDERS COMPAGNY, ORLANDO, FL, US, vol. 94, no. 10,SUPPL01PT01, 15 November 1999 (1999-11-15), page 221A XP001024606 ISSN: 0006-4971
- FELDMAN M ET AL: "Anti-TNFalpha therapy is useful in rheumatoid arthritis and Crohn's disease: analysis of the mechanism of action predicts utility in other diseases" TRANSPLANTATION PROCEEDINGS, ORLANDO, FL, US, vol. 30, no. 8, 1 December 1998 (1998-12-01), pages 4126-4127, XP002098301 ISSN: 0041-1345
- SCHAEFFER E M ET AL: "Tec family kinases in lymphocyte signaling and function" CURRENT OPINION IN IMMUNOLOGY, CURRENT BIOLOGY LTD, XX, vol. 12, no. 3, 1 June 2000 (2000-06-01), pages 282-288, XP004257675 ISSN: 0952-7915

## Description

The invention relates to the use of inhibitors of the Tec family of protein tyrosine kinases to treat a condition. Typically the condition is associated with cytokine production, particularly TNF and IL-1β production. The condition may be sepsis, and/or septic shock.

Toll-like receptors (TLRs) are a group of germline-encoded receptors known in the art (Rock *et al* (1998) *PNAS* 95, 588-593). 9 TLRs are currently known (Du *et al* (2000) *Eur Cytokine Netw* 11, 362-371) and many more expected to exist.

Although the extracellular portions of Toll-related receptors (TRRs), including TLRs, IL-1R and IL-18R, are relatively divergent, the cytoplasmic portions are more conserved: They contain a well-defined region known as the toll domain, which is also found in the cytoplasmic portion of proteins comprising the IL-1 receptor, the IL-18 receptor and other receptors broadly termed the IL-1 receptor family. In addition, soluble cytoplasmic proteins such as MyD88 can have Toll domains. TLRs and IL-1 receptoir use an analogous framework of signalling; upon ligand binding, they recruit the adaptor molecule MyD88 through homotypic interactions with a toll domain that MyD88 contains in its C-terminus. MyD88, in turn, recruits IRAK, TRAF-6 and TollIP to activate NF-κB and mitogen-activated protein kinases (O'Neill & Dinarello (2000) *Immunol Today* **21,** 206-209; Burns *et al* (2000) *Nature cell Biol*. **2,** 346-351).

The MyD88 (myeloid differentiation protein) is considered to have a modular organisation consisting of an N-terminal death domain (DD) separated by a short linker from a C-terminal Toll domain (reviewed in Bums *et al* (1998) *J Biol Chem* **273,** 12203-12209). The N-terminal DD is related to a motif of approximately 90 amino acids that is considered to mediate protein-protein interactions with other DD sequences forming either homo- or heterodimers (Boldin *et al* (1995) *J Biol Chem* **270,** 387-391).

The MyD88 Toll domain has about 130 amino acids *(Mitcham et al* (1996) *J Biol Chem* **199,** 144-146). Toll domains are also considered to mediate protein-protein interactions with other Toll domains forming either homo- or heterodimers (see Bums *et al* (1998) *J Biol Chem* **273,** 12203-12209).

DD and Toll-Toll interactions are considered to be involved in directing signalling pathways. MyD88 is considered to bind via its Toll domain to TLRs and the IL-1 receptor (when bound to ligand). In turn, MyD88 is considered to bind *via* its DD to other DD-containing proteins; in particular it is considered to bind to IRAK and TRAF-6, thereby activating NF-κB and mitogen-activated protein kinases (O'Neill & Dinarello (2000) *Immunol Today* **21,** 206-209).

TRRs include components of the mammalian anti-microbial response (Wyllie *et al*. (2000) *J. Immunol*. **165**(12), 7125-7132) and many TRR ligands are known in the art. Individual TLRs are known to activate, *inter alia,* specialised anti-fungal or anti-bacterial genes through the activation of the NF-κB transcription factors (O'Neill & Dinarello (2000) *Immunol Today* **21,** 206-209).

TLRs, possibly in combination with IL-1R, are known to bind bacterial DNA (CpG-DNA) to intiate innate defense mechanisms against infectious pathogens *in vivo* (Hacker *et al*. (2000) *J. Exp. Med*., **192**(4), 595-600).

TLR2 has been demonstrated to bind microbial ligands *(Hertz et al*. (2001) *J. Immunol*., **164**(4), 2444-2450), factors from Gram positive bacteria (Takeuchi *et al*. (2000) *J. Immunol*. **165**(10), 5392-5396), reactive oxygen species (ROS) produced during oxidative stress (Frantz *et al., J. Biol. Chem.,* 16 November 2000), lipoteichoic acid, lipopeptides, glycans and, in combination with TLR6, modulin secreted from Gram positive bacteria such as Staphylococcus (Ozinsky *et al*. (2000) *Proc. Natl. Acad. Sci. USA*, **97**(25), 13766-13771; Hajjar *et al*. (2001) *J. Immunol*., **166**(1), 15-19) and other factors from *Staphylococcus arueus* bacteria (Takeuchi *et al*. (2000) *J. Immunol.* **165**(10), 5392-5396). TLR2 confers responsiveness to bacterial peptidoglycan and lipoteichoic acid as well as yeast carbohydrates such as the yeast cell-wall particle zymosan. This suggests that TLR2 principally mediates signals from yeast and Gram-positive bacteria (Underhill *et al* (1999) *Nature* **401,** 811-815), possibly as a functional pair with TLR6 or TLR1 (Ozinsky *et al*. (2000) *Proc. Natl. Acad. Sci. USA*, **97**(25), 13766-13771). Macrophage phagocytosis of zymosan is accompanied by secretion of the inflammatory mediator tumour necrosis factor-α (TNF-α). However, the signalling pathway that elicits TNF-α production is unknown (Underhill *et al* (1999) *Nature* **401,** 811-815).

TLR 1 has been shown, possibly as a dimer with TLR2, to bind soluble factors from *Niesseria meingitidis* and LPS (possibly either complexed to other molecules) (Wyllie *et al*. (2000) *J. Immunol.* **165**(12), 7125-7132).

Endogenous lumenal bacterial flora, which can be responsible for irritable bowel disease (IBD) including Crohn's disease and ulcerative colitis, have been shown to act due to changes in the regulation of expression of TLR2, TLR3, TLR4 and TLR5 (Cario & Porolsky (2000) *Infect. Immun*., **68**(12), 7010-7017). TLR 4 acts as a receptor for bacterial lipopolysaccharide (LPS), such as Escherichia and Salmonella LPS (Poltorak *et al* (1998) *Science* **282,** 2085-2088; *Tapping et al*. (2000) *J. Immunol*. **165**(10), 5780-5787) and has been shown to confer responsiveness to Gram negative bacteria (Underhill *et al* (1999) *Nature* **401,** 811-815. Data suggests that at least some LPS may act through TLR4 in association with CD14 (Schroder *et al*. (2000) *J. Immunol*. **165**(5), 2683-2693) or in association with CD14 and CD11b/CD18 (Perera *et* *al*. (2001) *J. Immunol*. **166**(1), 574-581). The LPS mimetic Taxol has also been shown to act through TLR4 and CD18 (Perera *et al*. (2001) *J. Immunol*. **166**(1), 574-581). The TLR RP105 has also been implicated in LPS signalling in cooperation with TLR4 in B cells (Ogata *et al*. (2000) *J. Exp. Med,* **192**(1), 23-29). TLR4 has also been shown to bind the EDA domain of fibronectin, fragments of which have been implicated in physiological and pathological processes, especially tissue remodeling associated with inflammation, such as rheumatois arthritis (RA).

Lipopolysaccharides (LPS) are the principle biochemical constituents of the external covering which characterises all Gram-negative bacteria. LPS play a major pathogenic pathophysiologic and immunologic role in Gram-negative infections. They are a major cause of the physiological effects associated with sepsis and septic shock. Accordingly, being able to inhibit the LPS-induced inflammatory pathway is important.

LPS stimulates immune responses by interacting with the membrane receptor, CD 14, possibly associated with toll-like receptors 2 and 4, to induce the generation of cytokines such as tumour necrosis factor-α (TNF-α), interleukin-1 (IL-1) and interleukin-6 (IL-6). Cytokines such as TNF, interleukins 1-18 and transforming growth factors (TGF) act in a complex interacting network on leucocytes, vascular endothelial cells, mast cells, haemopoietic stem cells, fibroblast and osteoclasts, controlling proliferation, differentiation and/or activation through autocrine or paracrine mechanisms. Several cytokines, for example TNF-α and IL-1 are important inflammatory mediators and have been implicated in several chronic inflammatory and autoimmune diseases such as rheumatoid arthritis, Crohn's disease and sepsis. The cytokines are being increasingly studied because of their importance in the formation of inflammatory and autoimmune diseases.

At present the mechanism by which the LPS signal is transduced from the extracellular membrane to the nucleus is not fully elucidated. One of the earliest signals detected in response to LPS binding to its receptor, CD14, is the activation of protein tyrosine kinases (PTKs), in particular the Src-family kinases p56lyn, p58hck and p59c-fgr (Stefanova *et al.* (1993) *J. Biol. Chem*. **268**(28), 20725-20728; Beaty *et al*. (1994) *Eur. J. Immunol*., **24,** 1278-1284; Herrera-Velti & Reiner (1996), *Am. Associat. Immunol*., **156,** 1157-1165). In a study using human monocytes it has been shown that LPS rapidly activates CD14-associated p56lyn simultaneously with PTKs p58hck and p59c-fgr (Stefanova *et al*. (1993) *J. Biol. Chem*. **268**(28), 20725-20728). Furthermore, Stefanova *et al.* (1993, *J. Biol. Chem*. **268**(28), 20725-20728) demonstrated that inhibition of PTKs with herbimycin A completely blocks LPS-induced production of TNF-α and IL-1. These results thus suggest a critical role for PTKs in the LPS/CD14-mediated signal transduction pathway in human monocytes. Moreover, Geng *et al*. (1993, *J. Immunol*., **151,** 6692-6700) used genistein and herbimycin A to block LPS-induced TNF production. However, no attempt was made to identify any tyrosine kinases. Novogradsky *et al.* (1994, *Science,* **264,** 1319-1322) also used an inhibitor, tyrophostin AG126 to block TNF production, but again did not identify any tyrosine kinases involved.

However, a more recent genetic approach (Meng & Lowell (1997) *J. Exp. Med*., **185,** 1661-1670) has shown that despite null mutations to lyn, hck, and fgr in a single mouse strain, there was no major defects in LPS signalling including TNF-α, IL-1 and IL-6 production (although the total protein phosphotyrosine level was greatly reduced in macrophages derived from these triple mutant mice). The data provided evidence that the three Src-family kinases p56 lyn, p58 hck and p59 c-fgr are not obligitory for LPS-initiated signal transduction.

Bruton's tyrosine kinase (Btk), a member of the Tec family of PTKs, is a non-receptor tyrosine kinase that has been shown to involved in B-cell development and B cell receptor (BCR) signalling. Mutations in the Btk gene leads to the condition known as X-linked agammaglobulinaemia (XLA) in humans and XID in mice.

XLA is the prototypical humoral immunodeficiency first described by Bruton in 1952 (Bruton (1952) *Pediatrics,* **9,** 722-727). It is characterised by a paucity of circulating B cells and a marked reduction in serum levels of all Ig isotypes, which causes susceptibility to recurrent and severe bacterial infections in affected males. The XID phenotype is exemplified by a milder immunodeficiency than XLA with reduced numbers of mature B cells and reduced serum levels of IgM and IgG3 (Perlmutter *et al*. (1979) *J. Exp. Med*., **149,** 993-998). The defect in XLA is considered to be due to inefficient expansion of pre-B cells into later B-cell stages or incomplete differentiation ot B-cell precursors to pre-B cells. In 1993, the gene responsible for XLA was identified as a cytoplasmic tyrosine kinase, named Bruton's tyrosine kinase (Btk). Btk belongs to a group of related cytoplasmic tyrosine kinases, known as the Tec family, and consists of five distinct structural domains, which encompass the N-terminus, pleckstrin homology (PH) domain, Tec homology (TH) domain (also known as the Btk domain), Src homology 3 (SH3) domain, SH2 domain and the catalytic kinase (SH1) domain. The Btk gene analysis has facilitated the identification of various mutations, including point mutations, insertions or deletions, in XLA cases. Mutations have been identified in all five domains of Btk and have been observed to be associated with a reduction in Btk mRNA, Btk protein, and kinase activity. Interestingly, the XID phenotype in mice is accounted for by a single point mutation within the PH domain of the murine gene.

B cells from xid mice have been shown to fail to proliferate in response to BCR cross-linking, by anti-Ig antibodies and are hyporesponsive to LPS (Amsbaugh *et al*. (1972) *J. Exp. Med*., **136,** 931-949; Huber & Melchers (1979) *Eur. J. Immunol*., **9,** 827-829; Scher (1982) *Adv. Immunol*., **33,** 1-71). Btk has also been implicated in upregulating nitric oxide production in xid mice macrophages in response to various stimuli, and the induction of macrophage effector cytokines is poorer in CBA/N than in CBA/J macrophages (Mukhopadhyay *et al*. (1999) *J. Immunol.,* **163,** 1786-1792). However, the expression of TNF-α in xid mice macrophages in response to LPS-stimulation has been demonstrated to be indistinguishable from the response of the wild type (Hata *et al*. (1998) *J. Exp. Med*., **187**(8), 1235-1247) thus implying that there is no requirement for Btk in LPS-stimulated TNF-α secretion. A similar result was achieved by *Zhao et al*. (1995, *J. Immunol*., **155,** 2067-2076) where it was demonstrated that , following stimulation with staphylococcal cell walls, there was no significant difference in TNF-α production between xid cells and wild type cells. Moreover, *Reid et al*. (1997, *J. Immunol*., **159,** 970-975) showed that Btk deficient mice are highly sensitive to endotoxin and have impaired clearance of LPS endotoxin. The ability to respond to endotoxin in Btk deficient would suggest that Btk is not a requirement for responding to bacterial toxins and so one would not expect inhibiting Btk to have an oppressive (detrimental) effect on LPS-induced sepsis.

Against this background, the inventors have attempted to identify signalling molecules that are required for expression of cytokines, such as TNF and IL-1β. They have found that members of the Tec family of PTKs are involved in mediating cytokine production. The Tec family PTKs have been demonstrated to play a role in upregulation of cytokine production in response challenges with to Toll-related receptor (TRR) ligands. The inventors have shown that Tec family PTKs are involved in the signal transduction pathway leading from LPS stimulation to TNF and IL-1β production. The inventors have also shown that Tec family PTKs are involved in the signal transduction pathway leading from zymosan stimulation to TNF production. Thus inhibitors of Tec family PTKs may be use to inhibit TRR-mediated signalling pathways leading to cytokine production. These results were unexpected, particularly in light of Hata *et al*. (1998, *J. Exp. Med.,* **187**(8), 1235-1247), Zhao *et al.* (1995, *J. Immunol*., **155,** 2067-2076) and *Reid et al*. (1997, *J. Immunol*., **159,** 970-975). The inventors have demonstrated that monocytes from patients deficient in Btk expression are severely compromised in the ability to produce both TNF-α and IL-1β in response to LPS. Moreover, different Tec family PTKs are likely to be essential to TRR ligand-induced expression of cytokine in different cell types. Inhibition of Btk result in a decrease in the levels of inflammation-associated factors such as TNF-α in monocytes, but Tec is a likely requirement in matured macrophages. Thus Tec family PTK inhibitors can be used to decrease cytokine production, such as TNF-α and IL-1β production, in response to a TRR ligand, such as LPS or zymosan. The inventors have developed means of reducing the effects of TRR signalling pathways. The effects of TRR ligands such as LPS and zymosan can be reduced by inhibition of Tec family PTKs. Inhibitors of Tec family PTKs can thus be used to ameliorate the effects of pathogens, such as Gram-negative bacteria, Gram positive bacteria, and yeast, on the host. Since inhibition of Tec family PTK can be used to regulate cytokine production, such inhibitors can be used to decrease the inflammatory response to disease, to decrease sepsis and septic shock, as well as addressing rheumatoid arthritis and Crohn's disease.

The invention also provides the use of an inhibitor of a member or members of the Tec family of PTKs in the manufacture of a medicament for use in the treatment of a condition associated with cytokine production, wherein said condition is rheumatoid arthritis or a condition resulting from an infection.

By "treating" a condition we include amelioration of the condition to a useful extent. We also include prophylactic treatment.

In one embodiment the cytokine is TNF, preferably TNFα. In another embodiment the cytokine is IL-1, preferably IL-1β. Typically the Tec family member is selected from Tec, Btk, Bmx, Txk or Itk, more typically the Tec family member is selected from Tec, Btk, Bmx or Txk, usually from Tec, Btk or Bmx. Preferably the Tec family member is Tec or Btk, most preferably Btk. In another embodiment the Tec family PTK is Tec. The cytokine-associated condition may be any one of sepsis, septic shock, or rheumatoid arthritis.

In a preferred embodiment the cytokine-associated condition is induced by a Toll-related receptor (TRR) ligand. In another preferred embodiment the cytokine-associated condition is induced by lipopolysaccharide (LPS). The condition may be induced by Gram-negative bacteria. The condition may be induced by Gram-positive bacteria. In another preferred embodiment the cytokine-associated condition is induced by zymosan. The condition may induced by yeast. The TRR ligand may be a microbial factor. The microbial factor may be from Staphylococcus, such as *Staphylococcus aureus*. The TRR ligand may be bacterial DNA, typically CpG-DNA. The TRR ligand may be a microbial ligand derived from a Gram positive bacteria. The TRR ligand may be a reactive oxygen species (ROS). The TRR ligand may be factor produced during' oxidative stress. The TRR ligand may be lipoteichoic acid. The TRR ligand may be a lipopeptide. The TRR ligand may be a glycan. The TRR ligand may be a yeast derived factor, usually a cell-wall particle, such as zymosan. The TRR ligand may be a factor from Niesseria meingitidis. The TRR ligand may be LPS. The TRR ligand may be LPS derived from Escherichia or Salmonella. The LPS may be complexed to other molecules. The TRR ligand may be an LPS mimetic, such as Taxol. The TRR ligand may be derived from endogenous lumenal bacterial flora. The TRR ligand may be fibronectin. The TRR ligand may be a fragment of fibronectin. The TRR ligand may be the EDA domain of fibronectin.

The invention also provides a method for identifying an inhibitor of a member or members of the Tec family of PTKs which inhibitor is suitable for use in a the treatment of a condition associated with cytokine production wherein said condition is rheumatoid arthritis or a condition resulting from an infection, the method comprising:
(a) providing, as a first component, a cell capable of TRR ligand-induced cytokine production;
(b) providing, as a second component, a TRR ligand;
(c) contacting the first and the second components in the presence of a test agent;
(d) determining whether the test agent is able to inhibit the TRR ligand-induced activity of a member of the Tec family of PTKs;
thereby to determine whether the test agent could act as an inhibitor of cytokine production. Typically the method further comprises the step of determining whether the inhibitor is specific for Tec family PTKs. Typically the Tec family member is selected from Tec, Btk, Bmx, Txk or Itk, more typically the Tec family member is selected from Tec, Btk, Bmx or Txk, usually from Tec, Btk or Bmx. Preferably the Tec family member is Tec or Btk, most preferably Btk. In another embodiment the Tec family PTK is Tec.

The invention also provides the use of an inhibitor of a member of the Tec family of PTKs to study the inhibition of sepsis, and/or septic shock caused by a TRR-ligand, preferably LPS, *in vitro*.

Disclosed herein is a method of treating sepsis or septic shock in a mammal administering a pharmaceutically effective amount of an inhibitor of Bruton's tyrosine kinase (Btk).

Disclosed herein is a Btk inhibitor for use as a medicament.

Also disclosed is a Btk inhibitor for use in the manufacture of a medicament to treat sepsis, septic shock and/or rheumatoid arthritis.

The invention provides a method or inhibitor of the invention wherein the inhibitor is LFM-A13.

The invention provides a method or inhibitor of the invention wherein the inhibitor is a vaccine, an antibody or a fragment thereof which is capable of binding Btk or a fragment thereof.

Disclosed herein is a method or inhibitor of the invention wherein the Btk inhibitor is antisense nucleic acid effective against Btk.

Also disclosed is a method or inhibitor of the invention wherein the inhibitor is inactivated Btk, or a fragment thereof.

Disclosed herein is the use of an inhibitor of Btk to study the inhibition of sepsis, and/or septic shock.

Also disclosed is a method of treating sepsis, and/or septic shock in a mammal comprising administering a pharmaceutically effective amount of an inhibitor of a member of the Tec family of protein tyrosine kinases (PTKs).

Further disclosed is a method of treating rheumatoid arthritis in a mammal comprising administering a pharmaceutically effective amount of an inhibitor of a member of the Tec family of protein tyrosine kinases (PTKs).

Disclosed herein is a method of treating a condition induced by a TRR ligand in a mammal comprising administering a pharmaceutically effective amount of an inhibitor of a member of the Tec family of protein tyrosine kinases (PTKs).

Also disclosed is a method of treating a condition induced by a TLR2 ligand in a mammal comprising administering a pharmaceutically effective amount of an inhibitor of a member of the Tec family of protein tyrosine kinases (PTKs).

Further disclosed is a method of treating a condition induced by a TLR4 ligand in a mammal comprising administering a pharmaceutically effective amount of an inhibitor of a member of the Tec family of protein tyrosine kinases (PTKs).

Disclosed herein is a method of treating a condition induced by a pathogen in a mammal comprising administering a pharmaceutically effective amount of an inhibitor of a member of the Tec family of protein tyrosine kinases (PTKs).

Also disclosed is a method of treating a condition induced by bacteria, or a factor derived therefrom in a mammal comprising administering a pharmaceutically effective amount of an inhibitor of a member of the Tec family of protein tyrosine kinases (PTKs).

Also disclosed is a method of treating a condition induced by Gram-negative bacteria, or a factor derived therefrom in a mammal comprising administering a pharmaceutically effective amount of an inhibitor of a member of the Tec family of protein tyrosine kinases (PTKs).

Also disclosed is a method of treating a condition induced by Gram-positive bacteria, or a factor derived therefrom in a mammal comprising administering a pharmaceutically effective amount of an inhibitor of a member of the Tec family of protein tyrosine kinases (PTKs).

Disclosed herein is a method of treating a condition induced by LPS, or an analogue thereof, such as Taxol, in a mammal comprising administering a pharmaceutically effective amount of an inhibitor of a member of the Tec family of protein tyrosine kinases (PTKs).

Also disclosed is a method of treating a condition induced by yeast, or a factor derived therefrom in a mammal comprising administering a pharmaceutically effective amount of an inhibitor of a member of the Tec family of protein tyrosine kinases (PTKs).

Disclosed herein is a method of treating a condition induced by zymosan, or an analogue thereof, in a mammal comprising administering a pharmaceutically effective amount of an inhibitor of a member of the Tec family of protein tyrosine kinases (PTKs).

Also disclosed is a method of treating a condition induced by fibronectin, or a fragment or variant thereof, in a mammal comprising administering a pharmaceutically effective amount of an inhibitor of a member of the Tec family of protein tyrosine kinases (PTKs).

The term cytokine is well known in the art and as used herein includes within its meaning interleukins such as IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22 and IL-23, tumour necrosis factors (TNF) such as TNFα and TNFβ (also known as lymphotoxin), interferons such as IFNα, IFNβ and IFNγ, colony stimulating factors such as M-CSF, G-CSF, GM-CSF, migration inhibition factor (MIF), chemokines such as IL-8, IP10 and VEGF. Particularly preferred cytokines are those produced by monocytes and macrophages, such as IL-1α, IL-1β, IL-6, TNFα, M-CSF, G-CSF and GM-CSF. In a preferred embodiment the cytokine is TNFα. In another preferred embodiment the cytokine is IL-1β.

As used herein, TNF refers to a type of cytokine well known in the art that is released by monocytes such as activated macrophages and is structurally related to lymphotoxin, which is released by activated T cells. In a preferred embodiment the TNF is TNF-α.

TRRs include molecules such as TLRs, IL-1 receptor family members including IL-1 receptor and IL-18 receptor and cytoplasmic proteins such as MyD88.

The term lipopolysaccharide (or LPS) is well known in the art and refers to the principle biochemical constituents of the external covering of Gram-negative bacteria. Typically an LPS will comprise three distinct regions joined by covalent linkages. The hydrophobic portion comprises a single region, termed lipid A, which is usually responsible for the toxic properties of LPS. The hydrophilic region consists of two regions: an O-specific repeating oligosaccharide; and a core oligosaccharide which links which links the O-side chain to the lipid A. The biological effects of LPS are diverse and have been documented as including fever, circulatory disturbances and vascular hyper-reactivity to adrenergic drugs, leucopaenia typically followed leucocytosis, non-specific stimulation of B-lymphocytes to undergo blast transformation and proliferation, lethal toxicity and non-specific tolerance to endotoxin through repeated exposure to LPS. Exposure to LPS is also known to cause an increase in the production of cytokines such as TNF.

Thus the present invention provides methods for reducing expression of cytokine, for example in response to a TRR ligand, and uses of inhibitors of Tec family PTKs. Specific inhibitors of the Tec family may be used in antiinflammatory therapeutics. Accordingly the inventors have shown that inhibitors of Tec family PTKs can be used to reduce the effects of infection by Gram negative bacteria. The types of bacterial infections that can be addressed by the methods and uses of the invention include, but are not limited to, salmonellae, brucellae, meningococci, gonococci, streptococci, *Haemophilus influenzae, Klebsiella pneumoniae* and non-enteric infections due to *E.coli*. An inhibitor of Tec family PTKs can be used to reduce the effects of LPS, thereby to ameliorate the effects of the bacterial infection. In one embodiment an inhibitor of Tec family PTKs can be used to reduce or otherwise ameliorate sepsis or septic shock. In another embodiment, an inhibitor of Tec family PTKs can be used to reduce or otherwise ameliorate inflammation. In another embodiment an inhibitor of Tec family PTKs can be used to combat rheumatoid arthritis.

Other conditions for which inhibitors of Tec family PTKs may be used in the treatment of include conditions in which cytokines are associated. Preferred cytokines are IL-1β and TNFα and conditions associated therewith include conditions associated with local inflammation, for example conditions of the joints, such as osteoarthritis, spondyloarthropathy, psoriatic arthritis or lyme arthritis, conditions of the lung such as severe steroid resistant asthma, sarcoid or other pulmonary fibrosis, conditions of the heart such as myocarditis, dilated cardiomyopathy or congestive cardiac failure, conditions of vessels such as atherosclerosis, unstable angina with high CRP/IL-6, or vasculitis (such as Wegeners and others), conditions caused by reperfusion injury such as infarction and stroke, conditions of the CNS such as multiple sclerosis, cerebral odema or Alzheimer's disease, conditions of the thyroid such as graves opthalmopathy, conditions of the skin such as psoriasis or contact snesitivity, conditions associated with transplant rejection such as of the kidney, heart, lung, liver or bone marrow, conditions of the liver such as acute alcoholic hepatitis or chronic progressive viral hepatitis, conditions associated with fibrosis such as post-operative fibrosis (eg after back surgery), conditions resulting from surgery such as where a bypass activates leucocytes (eg CABG). Other conditions for which inhibitors of Tec family PTKs may be used in the treatment of include conditions resulting from infections such as HIV, parasitic diseases (eg cachexia), erythema nodosum lepromatum, borreliosis (eg lime arthritis), or meningococcal septicaemia. Other conditions for which inhibitors of Tec family PTKs may be used in the treatment of include conditions associated with cancer, such as where the cancerous tissue produces or induces TNF production, such as breast cancer, cancers having TNFα adhesion eg ovarian cancer or colon cancer, or cancers involving TNFα dependent aromatase upregulation of estrogen production in periphery

Inhibitors of Tec family PTKs can be used to treat LPS-induced conditions in a host. The host is typically a mammal, preferably a human.

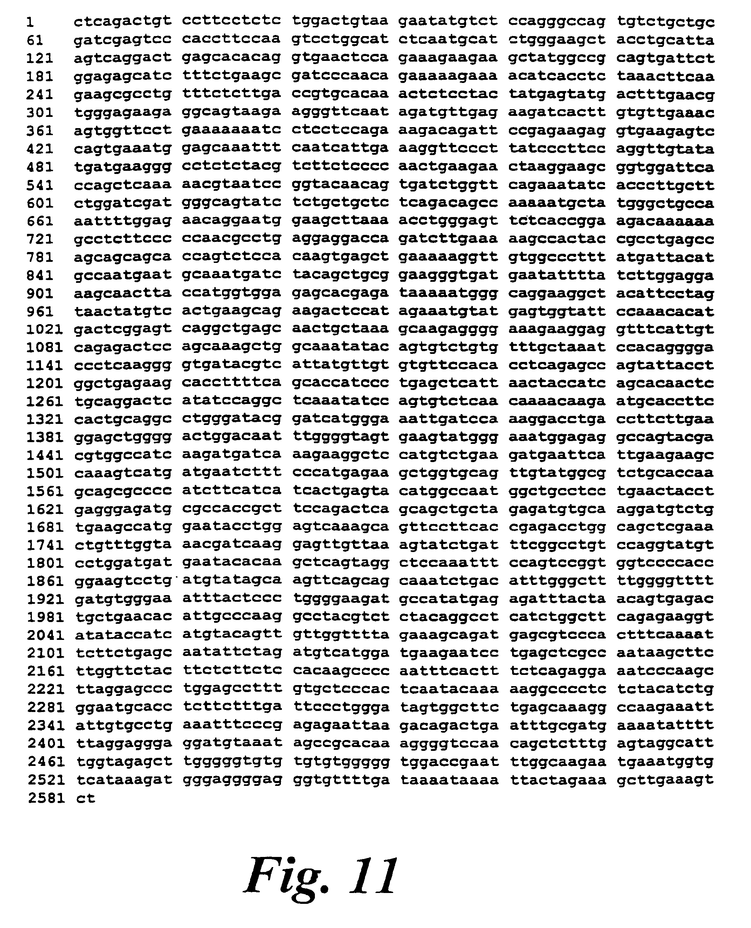

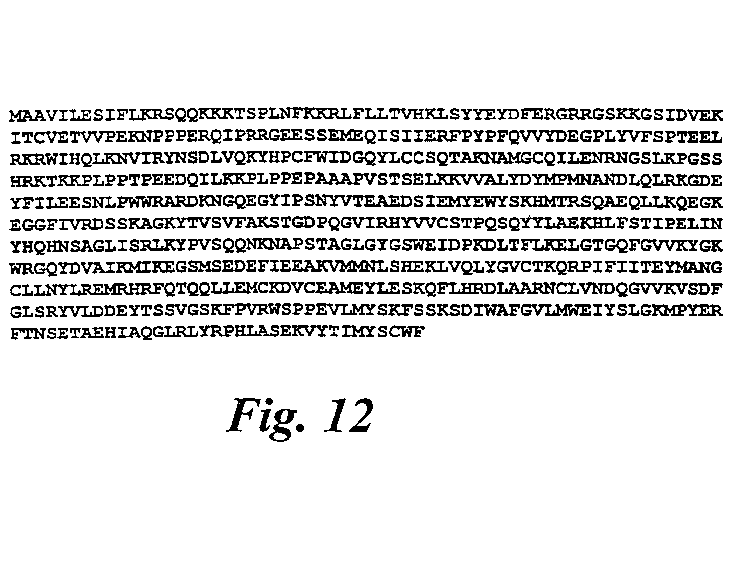

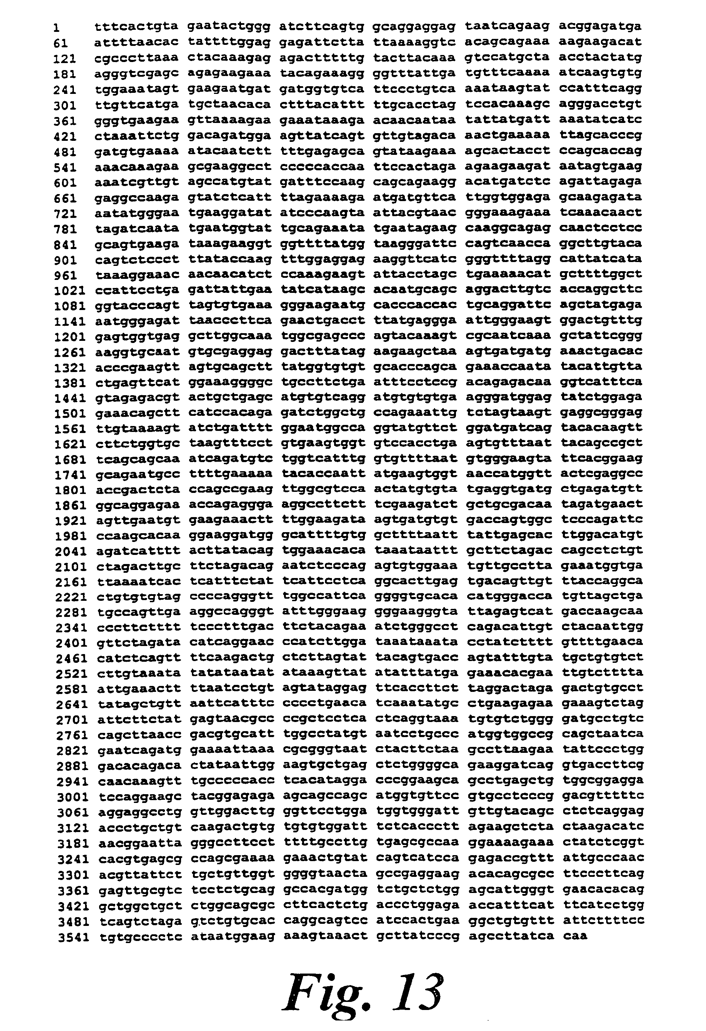

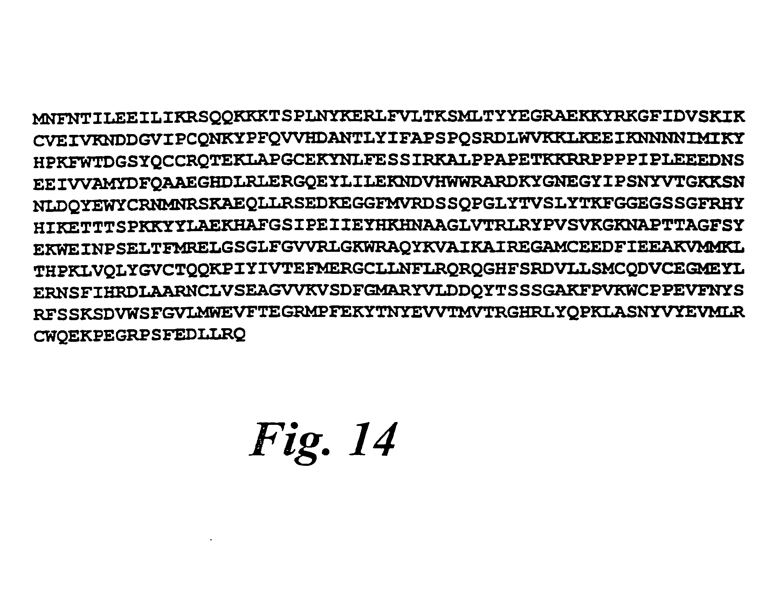

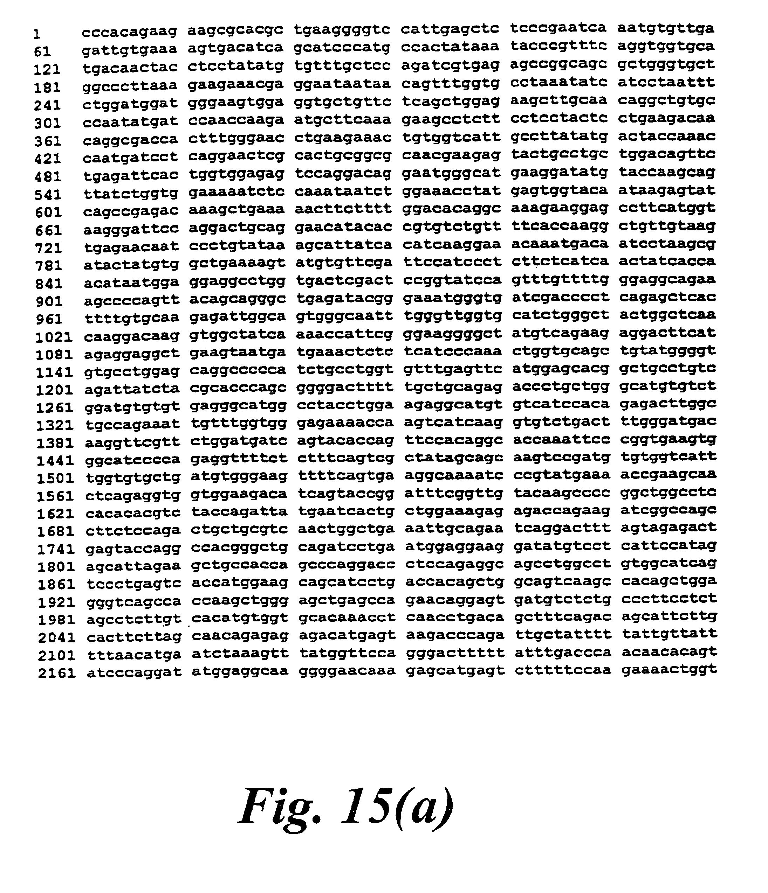

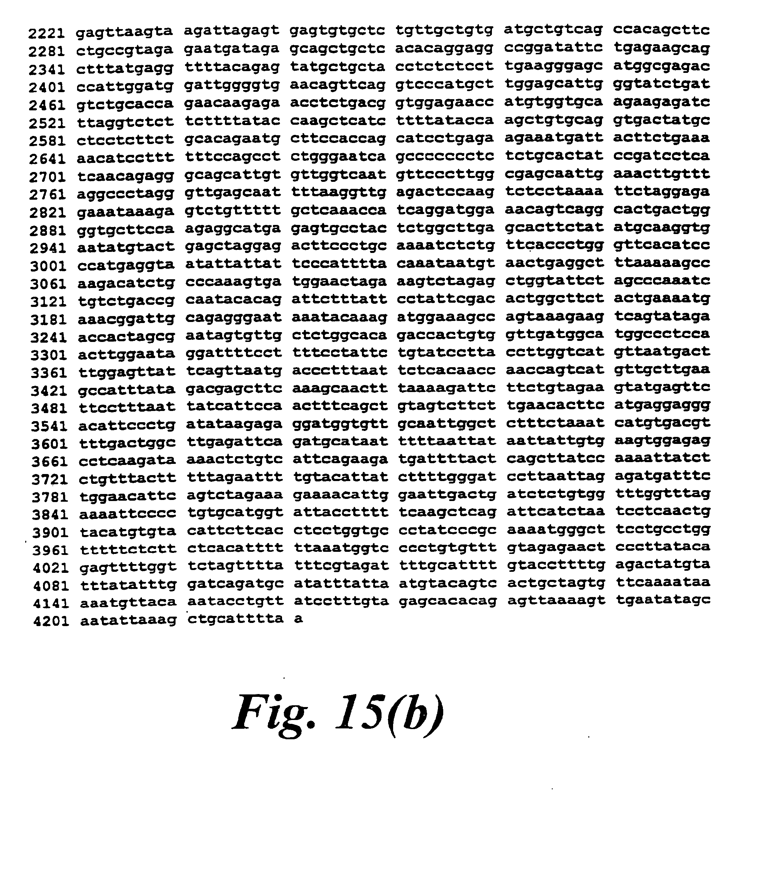

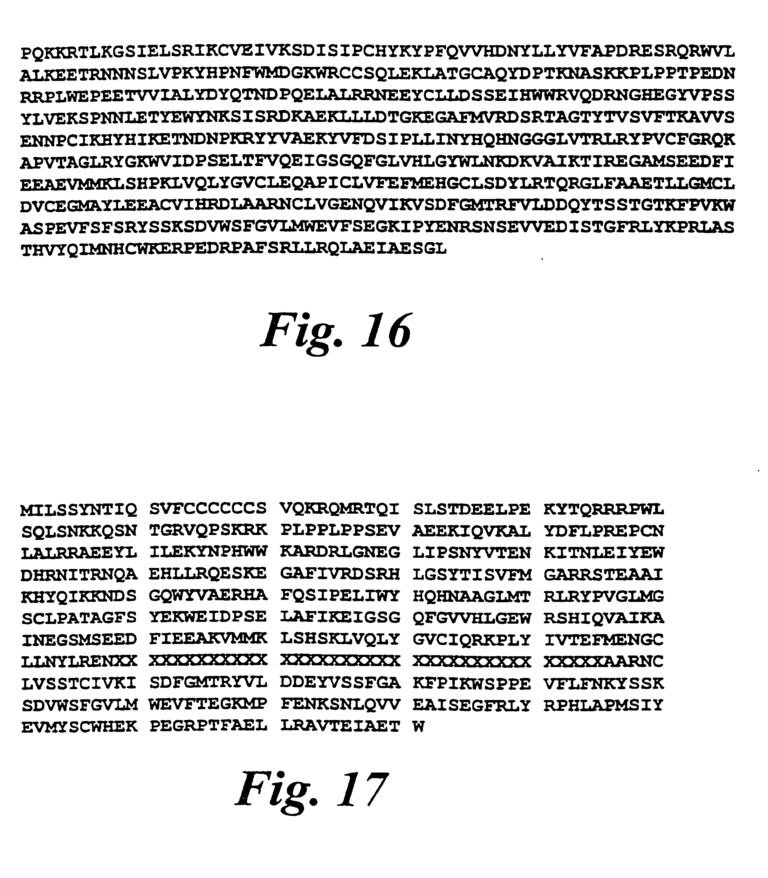

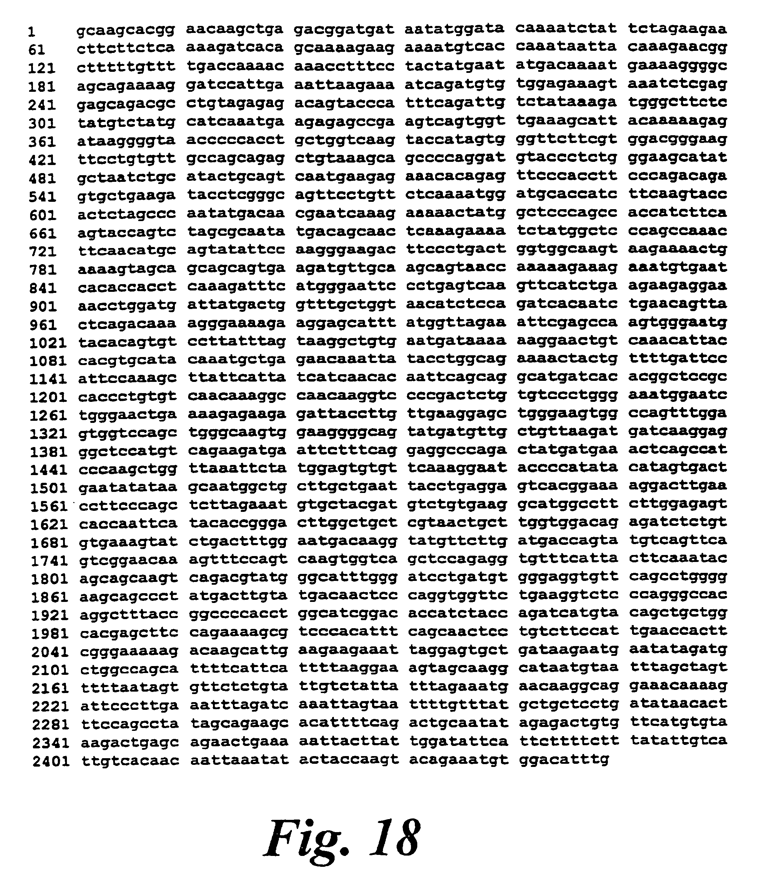

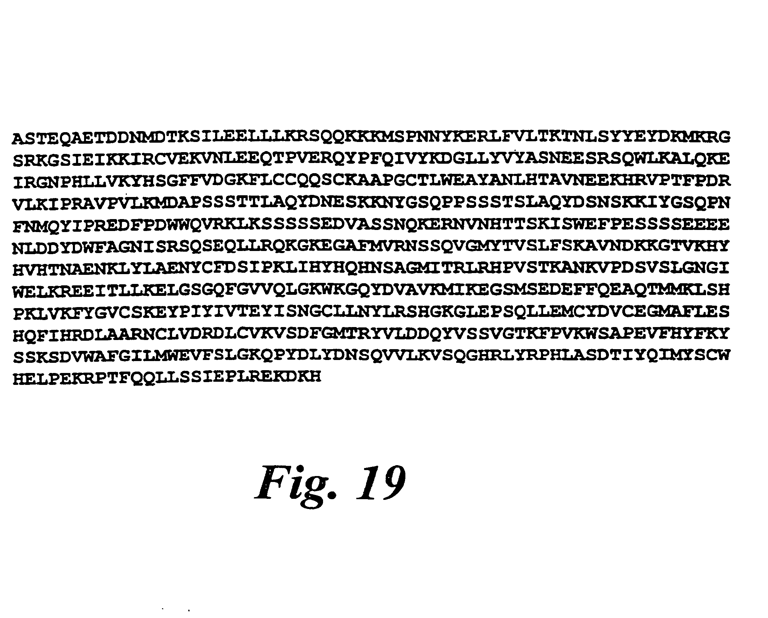

The Tec family of protein tyrosine kinases, also referred to as Tec family PTKs and Btk family kinases are a well defined group of non-receptor PTKs (Qiu & Kung (2000) *Oncogene,* **19,** 5651-5661; Schaeffer & Schwartzberg (2000) *Current Opinion in Immunology*, **12,**282-288; Mohamed *et al*. (1999) *Scand. J. Immunol.,* **49,** 113-118; Tomlinson *et al.* (1999) *J. Biol. Chem.,* **274**(19), 13577-13585). They include Bruton tyrosine kinase (Btk) *(Tsukada et al*. (1993) *Cell*, **72,** 279-290; *Vetrie et al*. (1993) *Nature,* **361,** 226-233) as defined in Figures 11 and 12, Tec (Sato *et al*. (1994) *Leukemia,* **8,** 1663-1672) as defined in Figures 13 and 14, Itk/Emt/Tsk (Siliciano *et al*. (1992) *Proc. Natl. Acad. Sci. USA*, **89,** 11194-11198) as defined in Figures 15 and 16, and Bmx/Etk *(Qiu et al*. (1998) *Proc. Natl. Acad. Sci. USA*, **95,** 3644-3649; Tamagnone *et al.* (1994) *Oncogene,* 9, 3683-3688) as defined in Figures 18 and 19 and Txk as defined in Figure 17. Other Tec family PTK members typically have at least 40%, usually from 50 to 60%, preferably more than 60% amino acid sequence identity to any one of Tec, Btk, Itk, Bmx or Txk (Tomlinson *et al*. (1999) *J. Biol. Chem*., **274**(19), 13577-13585).

Preferably, a member of the Tec family of PTKs will comprise at least one, more preferably two, more preferably three of the following domains selected from a kinase domain, an SH3 domain (Src Homology domain 3) and/or an SH2 domain (Src Homology domain 2) or a variant or fragment of both or either. The sequences of kinase, SH2 and SH3 domains are as defined by the translation products of the seqeunces in Figures 11, 13, 15 and 18 and as defined in Figure 17.

The kinase domain (also known as a Src Homology domain 1 (SH1 domain)) or a variant or fragment thereof is typically a C-terminal domain. By C-terminal we include the meaning that there are no domains present between the kinase domain and the C-terminus of the protein. Usually a C-terminal domain will end within 50 amino acids of the C-terminal of the protein, typically within 25 amino acids, more typically within 10 amino acids, preferably within 5 amino acids.

Typically a member of the Tec family of PTKs may include a Tec homology (TH) domain, for example between the PH and the SH3 domains. The TH domain comprises a proline rich motif and is able to bind to an SH3 domain. Typically the proline rich motif has the sequence PXPPXP or (R/K)XXPXXP (Qiu & Kung (2000) *Oncogene,* **19,** 5651-5661; Andreotti *et al.* (1997, *Nature,* **385,** 93-97). It is thought that the TH domain may interact with the SH3 domain (Andreotti *et al*. (1997) *Nature,* **385,** 93-97). This may be one means by which Tec family PTKs are regulated.

Typically a Tec family member will comprise an N-terminal PH (pleckstrin homology) domain (Qiu & Kung (2000) *Oncogene,* **19,** 5651-5661). By N-terminal domain we mean that there are no domains present between the PH domain and the N-terminus of the protein. Usually an N-terminal domain will begin within 50 amino acids of the N-terminal of the protein, typically within 25 amino acids, more typically within 10 amino acids, preferably within 5 amino acids.

By PH (pleckstrin homology) domain we include the meaning a sequence of amino acids as defined in Figures 11 (the PH domain of Btk) or Figure 13 (the PH domain of Tec), the PH domain of any other member of the Tec family of PTKs or a variant or fragment thereof. Typically, a PH domain of a Tec family PTK will bind phospholipids, heterotrimeric G-proteins, PKC isoforms, Stat 3, F-actin, Fas and FAK (Qiu & Kung (2000) *Oncogene,* **19,** 5651-5661).

Preferably a variant or fragment of a domain of a member of the Tec family of PTKs will have substantially the same biological activity, that is, the substantially same binding specificity and affinity, as the domain to which it relates in Btk or Tec. The biological activities of the domains of the Tec family ot PTKs are discussed at length in the prior art (Qiu & Kung (2000) *Oncogene,* 19, 5651-5661 incorporated herein by reference).

However, it should be understood that the defintion of Tec family kinases as used herein includes proteins that do not have one or more of the above domains. For example, Bmx shares little sequence homology with other Tec family PTKs within the proline-rich TH domain or the SH3 domain, whereas Txk is particularly atypical at the N-terminus, lacking a PH domain and a TH domain (Tomlinson *et al*. (1999) *J. Biol. Chem*., **274**(19), 13577-13585).

Preferably, inhibitors used in the invention are specific for a member or members of the Tec family of PTKs. By specific we include the meaning that the inhibitor can selectively cause a greater reduction in the activity of the member or members of the Tec family of PTKs than of other cellular protein tyrosine kinases. Preferably the reduction in Tec family PTK member activity will be up to 2-fold, more preferably up to 10-fold, yet more preferably up to 100-fold greater than reduction in activity of other cellular protein tyrosine kinases. Such 'other' protein tyrosine kinases include JAK1, JAK3, HCK, epidermal growth factor receptor kinase and insulin receptor kinase (Mahajan *et al*. (1999) *J. Biol. Chem.* **274,** 9587-9599). The skilled person will be well aware of methods known in the art for testing the effects of inhibitors on the activity of different tyrosine kinases.

Tec family PTK inhibitors may modulate the interaction between the SH3 domain of the Tec family PTK and a proline-rich region, either in the same molecule, e.g. in the TH domain, or in another molecule. An inhibitor may therefore block the interaction between SH3 domain and a proline-rich region.

Without being bound by theory, we believe that inhibitors of Tec family PTKs used in the invention may be able to reduce TNFα production by specific inhibition of the activity of a member or members of the Tec family of PTKs, particularly when the TNFα production is induced by LPS.

The inhibitor may be a chemical inhibitor. Any chemical capable of inhibiting Tec family PTKs may be used. Terreic acid has been demonstrated to inhibit Btk without affecting the activity of Lyn, Syk or MAP kinases and does not significantly affect IgE/antigen-induced tyrosine phosphorylation patterns (Kawakami *et al*. (1999) *Proc. Natl. Acad. Sci. USA* **96,** 2227-2232). A preferred chemical inhibitor is LFM-A13. LFM-A13 has previously been used as an anti-leukemic agent *(Mahajan et al*. (1999) *J. Biol. Chem*. **274,** 9587-9599; WO 99/54286) and has been demonstrated to specifically inhibit Btk in comparison to other non-Tec family PTKs.

The inhibitor may also be an antibody or a fragment thereof which is capable of binding a Tec family PTK member or a fragment of the Tec familt PTK member, preferably of binding Btk, or a fragment of Btk. Preferably binding of the antibody or a fragment thereof the to Tec family PTK member causes inhibition of the Tec family PTK member. Antibody fragments include Fab or (Fab)₂, or Fv which retains their anti-Tec family PTK member activity. The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison *et al* (1984) *Proc. Natl. Acad. Sci. USA* **81,** 6851-6855).

That antigenic specificity is conferred by variable domains and is independent of the constant domains is known from experiments involving the bacterial expression of antibody fragments, all containing one or more variable domains. These molecules include Fab-like molecules (Better *et al* (1988) *Science* **240,** 1041); Fv molecules (Skerra *et al* (1988) *Science* **240,** 1038); single-chain Fv (ScFv) molecules where the V_{H} and V_{L} partner domains are linked via a flexible oligopeptide (Bird *et al* (1988) *Science* 242, 423; Huston *et al* (1988) *Proc. Natl. Acad. Sci. USA* **85,** 5879) and single domain antibodies (dAbs) comprising isolated V domains (Ward *et al* (1989) *Nature* **341,** 544). A general review of the techniques involved in the synthesis of antibody fragments which retain their specific binding sites is to be found in Winter & Milstein (1991) *Nature* **349,** 293-299.

By "ScFv molecules" we mean molecules wherein the V_{H} and V_{L} partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining sites.

The antibody may, preferably, be a monoclonal antibody. With today's techniques monoclonal antibody antibodies can be prepared to most antigens. The antigen-binding portion may be a part of an antibody (for example a Fab fragment) or a synthetic antibody fragment (for example a single chain Fv fragment [ScFv]). Suitable monoclonal antibodies to selected antigens may be prepared by known techniques, for example those disclosed in *"Monoclonal Antibodies: A manual of techniques ",* H Zola (CRC Press, 1988) and in *"Monoclonal Hybridoma Antibodies: Techniques and Applications ",* J G R Hurrell (CRC Press, 1982).

Chimaeric antibodies are discussed by Neuberger *et al* (1988, *8th International Biotechnology Symposium* Part 2, 792-799).

Suitably prepared non-human antibodies can be "humanized" in known ways, for example by "grafting" the CDR regions of mouse antibodies into the framework of human antibodies.

The antibody may also be linked to polypeptide sequences that permit entry into cells. Such sequences are known and include the penetration peptide and HIV sequences which have been engineered to permit entry into cells. Alternatively, cDNA encoding such antibodies could be delivered by a vector. For example an antibody molecule may be delivered by gene therapy using suitable vectors such as adenoviral vectors. Such vectors may include a regulatory region such as a promoter operatively linked to the gene encoding the antibody molecule. The promoter may be constitutive or may be tissue or cell-type specific, such as specific to monocytes, eg using the CD14 or CD36 promoters.

Alternatively, the inhibitor may be a vaccine raised against any suitable part of a Tec family PTK member.

Alternatively, the inhibitor may be an antisense nucleic acid polynucleotide that is capable of binding nucleic acid sequences encoding Tec family PTKs, particularly Btk. The use of antisense nucleic acid to inhibit the translation or transcription of sequences encoding proteins is known in the art. Typically the polynucleotide is able to reduce Tec family PTK activity. The polynucleotide may be DNA or RNA and may comprise non-natural nucleotides. Preferably the polynucleotide will have direct or indirect antisense activity.

A polynucleotide with indirect antisense activity is a polynucleotide that, following its introduction into a host cell, can interact with the cellular components and cause the production of a polynucleotide having direct antisense activity, e.g. plasmids or retroviruses or other vectors carrying an antisense gene. This approach is typically termed antisense gene therapy.

Polynucleotides with direct antisense activity are single-stranded nucleic acids, which can specifically bind to a target nucleic acid sequence. The target nucleic acid sequence is a gene or genes encoding a member or members of the Tec family of PTKs. In one embodiment a polynucleotide having direct antisense activity binds to the gene for Btk (as defined in Figure 11). In another embodiment a polynucleotide having direct antisense activity binds to the gene for Tec (as defined in Figure 13).

Typically a directly active antisense polynucleotide will be complementary to the nucleic acid sequence to which it is intended to bind. By binding to the appropriate target sequence, an RNA-RNA, a DNA-DNA, or RNA-DNA duplex is formed. These nucleic acids are often termed "antisense" because they are complementary to the sense or coding strand of the gene. Antisense oligonucleotides were first discovered to inhibit viral replication or expression in cell culture for Rous sarcoma virus, vesicular stomatitis virus, herpes simplex virus type 1, simian virus and influenza virus. Since then, inhibition of mRNA translation by antisense oligonucleotides has been studied extensively in cell-free systems including rabbit reticulocyte lysates and wheat germ extracts. Inhibition of viral function by antisense oligonucleotides has been demonstrated *in vitro* using oligonucleotides which were complementary to the AIDS HIV retrovirus RNA (Goodchild, J. 1988 "Inhibition of Human Immunodeficiency Virus Replication by Antisense Oligodeoxynucleotides", *Proc. Natl. Acad. Sci. (USA)* **85**(15), 5507-11). The Goodchild study showed that oligonucleotides that were most effective were complementary to the poly(A) signal; also effective were those targeted at the 5' end of the RNA, particularly the cap and 5' untranslated region, next to the primer binding site and at the primer binding site. The cap, 5' untranslated region, and poly(A) signal lie within the sequence repeated at the ends of retrovirus RNA (R region) and the oligonucleotides complementary to these may bind twice to the RNA.

Recently, formation of a triple helix has proven possible where the oligonucleotide is bound to a DNA duplex. It was found that oligonucleotides could recognise sequences in the major groove of the DNA double helix. A triple helix was formed thereby. This suggests that it is possible to synthesise a sequence-specific molecule which specifically binds double-stranded DNA via recognition of major groove hydrogen binding sites. The use of polnucleotides in the formation of a triple helix is included within the scope of uses of polynucleotides as antisense inhibitors.

By binding to the target nucleic acid, the above polynucleotides can inhibit the function of the target nucleic acid. This could, for example, be a result of blocking the transcription, processing, poly(A) addition, replication, translation, or promoting inhibitory mechanisms of the cells, such as promoting RNA degradations.

Typically, antisense polynucleotides are 15 to 35 bases in length. For example, 20-mer oligonucleotides have been shown to inhibit the expression of the epidermal growth factor receptor mRNA (Witters *et al, Breast Cancer Res Treat* 53:41-50 (1999)) and 25-mer oligonucleotides have been shown to decrease the expression of adrenocorticotropic hormone by greater than 90% *(Frankel et al, JNeurosurg* 91:261-7 (1999)). However, it is appreciated that it may be desirable to use oligonucleotides with lengths outside this range, for example 10, 11, 12, 13, or 14 bases, or 36, 37, 38, 39 or 40 bases. Thus the antisense polynucleotide may be up to 10, 25, 50, 75, 90, 95, 99 or 100% of the length of the target nucleic acid. To put it another way, where the target nucleic acid is a Tec family PTK member gene, the antisense polynucleotide may be equivalent in length to any of the above mentioned percentages of the total length of that gene.

Antisense polynucleotides may be prepared in the laboratory and then introduced into cells, for example by microinjection or uptake from the cell culture medium into the cells, or they are expressed in cells after transfection with a polynucleotide having indirect antisense activity, e.g. plasmids or retroviruses or other vectors carrying an antisense gene.

Polynucleotides are subject to being degraded or inactivated by cellular endogenous nucleases. To counter this problem, it is possible to use modified polynucleotides, e.g. having altered internucleotide linkages, in which the naturally occurring phosphodiester linkages have been replaced with another linkage. For example, Agrawal *et al* (1988) *Proc. Natl. Acad. Sci. USA* **85,** 7079-7083 showed increased inhibition in tissue culture of HIV-1 using polynucleotides phosphoramidates and phosphorothioates. Sarin *et al* (1988) *Proc. Natl. Acad. Sci. USA* **85,** 7448-7451 demonstrated increased inhibition of HIV-1 using oligonucleotide methylphosphonates. Agrawal *et al* (1989) *Proc. Natl. Acad. Sci. USA* **86,** 7790-7794 showed inhibition of HIV-1 replication in both early-infected and chronically infected cell cultures, using nucleotide sequence-specific oligonucleotide phosphorothioates. Leither *et al* (1990) *Proc. Natl. Acad. Sci. USA* **87,** 3430-3434 report inhibition in tissue culture of influenza virus replication by oligonucleotide phosphorothioates.

Polynucleotides having artificial linkages have been shown to be resistant to degradation *in vivo.* For example, Shaw *et al* (1991) in *Nucleic Acids Res.* **19,** 747-750, report that otherwise unmodified polynucleotides become more resistant to nucleases *in vivo* when they are blocked at the 3' end by certain capping structures and that uncapped polynucleotides phosphorothioates are not degraded *in vivo.*

A detailed description of the H-phosphonate approach to synthesizing polynucleoside phosphorothioates is provided in Agrawal and Tang (1990) *Tetrahedron Letters* **31,** 7541-7544, the teachings of which are incorporated herein by reference. Syntheses of polynucleoside methylphosphonates, phosphorodithioates, phosphoramidates, phosphate esters, bridged phosphoramidates and bridge phosphorothioates are known in the art. See, for example, Agrawal and Goodchild (1987) *Tetrahedron Letters* **28,** 3539; Nielsen *et al* (1988) *Tetrahedron Letters* **29,** 2911; Jager *et al* (1988) *Biochemistry* **27,** 7237; *Uznanski et al* (1987) *Tetrahedron Letters* **28,** 3401; Bannwarth (1988) *Helv. Chim. Acta.* **71,** 1517; Crosstick and Vyle (1989) *Tetrahedron Letters* **30,** 4693; *Agrawal et al* (1990) *Proc. Natl. Acad. Sci. USA* **87,** 1401-1405, the teachings of which are incorporated herein by reference. Other methods for synthesis or production also are possible. In a preferred embodiment the polynucleotides is a deoxyribonucleic acid (DNA), although ribonucleic acid (RNA) sequences may also be synthesized and applied.

The polynucleotides useful in the invention preferably are designed to resist degradation by endogenous nucleolytic enzymes. *In vivo* degradation of polynucleotides produces polynucleotides breakdown products of reduced length. Such breakdown products are more likely to engage in non-specific hybridization and are less likely to be effective, relative to their full-length counterparts. Thus, it is desirable to use polynucleotides that are resistant to degradation in the body and which are able to reach the targeted cells. The present polynucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are known in the art. See, for example, Cohen, (1990) *Trends in Biotechnology*. The synthesis of polynucleotides having one or more of these linkages substituted for the phosphodiester internucleotide linkages is well known in the art, including synthetic pathways for producing polynucleotides having mixed internucleotide linkages.

Polynucleotides can be made resistant to extension by endogenous enzymes by "capping" or incorporating similar groups on the 5' or 3' terminal nucleotides. A reagent for capping is commercially available as Amino-Link II™ from Applied BioSystems Inc, Foster City, CA. Methods for capping are described, for example, by *Shaw et al* (1991) *Nucleic Acids Res.* **19,** 747-750 and Agrawal *et al (1991) Proc. Natl. Acad. Sci. USA* **88**(17), 7595-7599, the teachings of which are hereby incorporated herein by reference.

A further method of making polynucleotides resistant to nuclease attack is for them to be "self-stabilized" as described by *Tang et al* (1993) *Nucl. Acids Res.* **21,** 2729-2735 incorporated herein by reference. Self-stabilized polynucleotides have hairpin loop structures at their 3' ends, and show increased resistance to degradation by snake venom phosphodiesterase, DNA polymerase I and fetal bovine serum. The self-stabilized region of the polynucleotides does not interfere in hybridization with complementary nucleic acids, and pharmacokinetic and stability studies in mice have shown increased *in vivo* persistence of self-stabilized oligonucleotides with respect to their linear counterparts.

Polynucleotides with direct antisense activity are most preferably 100% complementary to the sequence of the target nucleic acid. However, the skilled person will appreciate that polynucleotides having less than 100% complementarity with the target nucleic acid can also be used. For example, polynucleotides having no less than 99%, 95%, 90%, 80%, 75%, or 50% complementarity with the target nucleic acid may be used to reduce the expression of a member or members of the Tec family of PTKs.

The term "vector" means a DNA molecule comprising a single strand, double strand, circular or supercoiled DNA. Suitable vectors include retroviruses, adenoviruses, adeno-associated viruses, pox viruses and bacterial plasmids Vectors useful for the expression of antibody-encoding genes include prokaryotic and eukaryotic expression vectors.

Typical prokaryotic vector plasmids are: pUC18, pUC19, pBR322 and pBR329 available from Biorad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

Vectors useful for providing a polynucleotide having indirect antisense activity, i.e. delivery vehicle for an antisense gene therapy approach, are typically mammalian cell vector plasmids.

Methods well known to those skilled in the art can be used to construct expression vectors containing the coding sequence of the desired gene, e.g. a gene encoding an anti-Tec family PTK member antibody, or an anti-Tec family PTK member antisense gene and, for example appropriate transcriptional or translational controls.

The transcriptional controls may be tissue or cell-type specific, thus enabling the expression of the anti-Tec family PTK member antisense gene in selected tissues or cells. For example, the transcriptional controls used may be specific for expression in monocytes, such as regulatory sequences, for example promoters, from CD 14 or CD36 genes. Alternatively the transcriptional controls may be constitutively active to enable the production of gene product in any type of transformed cell.

The inhibitor may also be inactivated Tec family PTK, such as Btk or a fragment thereof which is capable of competing with the naturally occurring form for Tec family PTK-binding sites, such as Btk-binding sites. Such inhibitors may be capable as acting as a dominant negative mutant would. This type of inhibitor results in a reduction in the activity of the Tec family PTK, such as Btk kinase. For example mutants Tec family PTKs can be generated by deletion or mutation of the kinase domain using techniques well known in the art, for example as described in Yamashita *et al*. (1998, *Blood,* **91**(5), 1496-1507). Mutant proteins can be produced by any method known in the art, such as by recombinant expression. The thus produced protein can then be administered to a patient. Alternatively, a polynucleotide encoding the mutant protein can be used as a delivery vehicle, such as by gene therapy, using methods discussed above.

The invention further anticipates a method of identifying agents that may be used as specific inhibitors of Tec family PTKs. Typically agents are initially screened to determine whether they can modulate Tec family PTK-associates activity. By modulate we mean that the agent can increase or decrease the activity of a Tec family PTK. Agents thus identified are usually then tested to determine whether they inhibit a member of members of the Tec family of PTKs specifically, that is to say, they inhibit selectively as discussed above.

In one embodiment the initial stage of the screen can be performed by determining the ability of a test agent to alter the production of a cytokine, such as TNF or IL-1β, in response to a stimulus. In one embodiment the stimulus is a TRR-ligand. In another embodiment the stimulus is LPS. In another embodiment the stimulus is zymosan. For example, a test cell can be incubated with a test agent for sufficient time to allow inhibition of Tec family PTKs. The cell is typically a monocyte, or a macrophage that has been matured from a monocyte using M-CSF. The concentration of test agent used is typically from 0.1pM to 1M, more typically from 0.1µM to 10mM, most typically from 1µM to 1mM. The length of incubation is usually no more than 24 hours, more typically no more than 12 hours, 6 hours, 4 hours, 2 hours or 1 hour. Preferably the inhibitor is effective after 30 minutes of incubation or less. The cell can then be challenged with the stimulus. The concentration of stimulus used is typically from 0.1ng/ml to 1µg/ml, preferably from 10ng/ml to 100ng/ml. Following the challenge with the stimulus, the production of cytokine can be measured using techniques well known in the art, such as an enzyme-linked immunosorbent assay (ELISA) as discussed in the methods section below or using a reporter gene construct having a cytokine promoter operably linked to a reporter gene such as luciferase or β-galactosidase and provided either as a stable integration in the genome of the test cell or by a vector that is transformed in to the test cell such as using an adenovirus vector. Test agents which cause aberrant levels of stimulus-stimulated cytokine production, that is, higher or lower levels of cytokine production than that of cells stimulated by the stimulus in the absence of the test agent, are identified and can be characterised further. Typically the further characterisation involves testing the thus identified agent to determine whether its action is specific for a member or members of the Tec family of PTKs. Methods for doing so are discussed below.

An alternative embodiment for the initial stage of the screen, or a method of further characterising inhibitors identified as causing aberrant stimulus-induced expression of cytokine (e.g. using a method as discussed above), is to determine the ability of the test agent to alter the stimulus-induced activity of Tec family PTKs. This can be achieved by methods well known in the art, for example by *in vivo* or *in vitro* tests.

Typically, in an *in vivo* test, a cell is incubated with a test agent, using conditions as discussed above, and then the cell is challenged with a stimulus. Following the challenge, the activity of Tec family kinases can be determined. For example, the levels of autophosphorylation may be indicative of Tec family PTK activity. This can determined by methods well known in the art, for example as discussed in the present application with respect to Btk and Tec. Alternatively the ability of isolated Tec family PTK to phosphorylate a tyrosine-containing substrate can be assayed in the presence and absence of the test agent.

Typically, in *an in vitro* test, a test agent is contacted with Tec family PTK polypeptide *in vitro* and it is determined whether, for example, the enzymic activity or the level or autophosphorylation of the said polypeptide is changed compared to the activity of the said polypeptide in the absence of said test agent. It will be understood that it will be desirable to identify agents that may modulate the activity of the polypeptide *in vivo.* Thus it will be understood that reagents and conditions used in *the in vitro* method may be chosen such that the interactions between the said polypeptide and its substrate are substantially the same as *in vivo.*

Typically agents identified for their ability to alter the stimulus-induced activity of Tec family PTKs are further characterised by testing their ability to modulate stimulus-induced cytokine production as discussed above and/or for specificity of action for a member or members of the Tec family of PTKs as discussed below.

In one embodiment, the test agent decreases the activity of said polypeptide, in other words the agent thus identified is an inhibitor. For example, the test agent may bind substantially reversibly or substantially irreversibly to the active site of said polypeptide. In a further example, the test agent may bind to a portion of said polypeptide that is not the active site so as to interfere with the binding of the said polypeptide to its substrate. In a still further example, the agent may bind to a portion of said polypeptide so as to decrease said polypeptide's activity by an allosteric effect. For example, the test agent may bind to the kinase domain, the SH2 domain, the SH3 domain, the PH domain or the TH domain.

In a further embodiment, the test agent increases the activity of said polypeptide, in other words the thus identified agent is an stimulator. For example, the test agent may bind to a portion of said polypeptide that is not the active site so as to aid the binding of the said polypeptide to its substrate. In a still further example, the test agent may bind to a portion of said polypeptide so as to increase said polypeptide's activity by an allosteric effect.

In an alternative initial screening method suitable for chemical inhibitors, the ability of a chemical test agent to bind to a Tec family PTK can be determined, typically followed by later tests for the ability of the chemical test agent to affect stimulus-induced cytokine production and/or to modulate Tec family PTK as described above, and usually followed by tests for the specificity of effect as described below. It will be appreciated that screening assays for the binding of an agent to Tec family PTK which are capable of high throughput operation will be particularly preferred. For example, an assay for identifying a chemical agent capable of modulating the activity of a protein kinase may be performed as follows. Beads comprising scintillant and a polypeptide that may be phosphorylated may be prepared. The beads may be mixed with a sample comprising the protein kinase and ³²P-ATP or ³³P-ATP and with the chemical test agent. Conveniently this is done in a 96-well format. The plate is then counted using a suitable scintillation counter, using known parameters for ³²P or ³³P SPA assays. Only ³²P or ³³P that is in proximity to the scintillant, i.e. only that bound to the polypeptide, is detected. Variants of such an assay, for example in which the polypeptide is immobilised on the scintillant beads via binding to an antibody, may also be used.

A further method of identifying a test agent that is capable of binding to a Tec family PTK is one where the Tec family PTK is exposed to the chemical test agent and any binding of the chemical test agent to the said Tec family PTK is detected and/or measured. The binding constant for the binding of the chemical test agent to the Tec family PTK may be determined. Suitable methods for detecting and/or measuring (quantifying) the binding of a chemical test agent to a Tec family PTK are well known to those skilled in the art and may be performed, for example, using a method capable of high throughput operation, for example a chip-based method. New technology, called VLSIPS™, has enabled the production of extremely small chips that contain hundreds of thousands or more of different molecular probes. These chips or arrays have probes arranged in arrays, each probe assigned a specific location. Chips have been produced in which each location has a scale of, for example, ten microns. The chips can be used to determine whether target molecules interact with any of the probes on the chip. After exposing the array to target molecules under selected test conditions, scanning devices can examine each location in the array and determine whether a target molecule has interacted with the probe at that location.

Once a test agent has been demonstrated to modulate, preferably to inhibit, Tec family PTK activity, and/or to modulate stimulus-induced cytokine production, its specificity may be determined by assaying its ability to modulate, preferably to inhibit, the activity of other protein tyrosine kinases, such as JAK1, JAK3, HCK, epidermal growth factor receptor kinase and insulin receptor kinase *(Mahajan et al*. (1999) *J. Biol. Chem*. **274,** 9587-9599). The skilled person will be well aware of methods known in the art for testing the effects of modulators, such as inhibitors, on the activity of different tyrosine kinases.

The methods described above include the screening of drugs or lead compounds. Thus, it will be appreciated that in the methods of screening test agents as described herein, the test agent may be a drug-like compound or lead compound for the development of a drug-like compound.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons and which may be watersoluble. A drug-like compound may additionally exhibit features of selective interaction with a particular protein or proteins and be bioavailable and/or able to penetrate target cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, poorly soluble, difficult to synthesise or has poor bioavailability) may provide a starting-point for the design of other compounds that may have more desirable characteristics.

Agents, preferably inhibitors, for use in the invention and identified by methods of the invention can be formulated with a pharmaceutically acceptable carrier to form a pharmaceutical formulation. Such formulations may conveniently be presented in unit dosage form and may be prepared by any of the methods well known in the art of pharmacy. Such methods include the step of bringing into association the active ingredient (e.g. an inhibitor used by or identified with a method of the invention) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The following example illustrate a pharmaceutical formulation according to the invention in which the active ingredient is a compound of any of the above structures.

### Injectable Formulation

Active ingredient 0.200 g
Sterile, pyrogen free phosphate buffer (pH7.0) to 10 ml

The active ingredient is dissolved in most of the phosphate buffer (35-40°C), then made up to volume and filtered through a sterile micropore filter into a sterile 10 ml amber glass vial (type 1) and sealed with sterile closures and overseals.

Salts which may be conveniently used in therapy include physiologically acceptable base salts, for example, derived from an appropriate base, such as an alkali metal (eg sodium), alkaline earth metal (eg magnesium) salts, ammonium and NX₄⁺ (wherein X is C₁₋₄ alkyl) salts. Physiologically acceptable acid salts include hydrochloride, sulphate, mesylate, besylate, phosphate and glutamate.

Salts according to the invention may be prepared in conventional manner, for example by reaction of the parent compound with an appropriate base to form the corresponding base salt, or with an appropriate acid to form the corresponding acid salt.

Thus, aforementioned compounds, agents and inhibitors of the invention or salts thereof can be used to treat the aforementioned conditions. The compounds, agents and inhibitors or a formulation thereof may be administered by any conventional method including oral and parenteral (eg subcutaneous or intramuscular) injection. The treatment may consist of a single dose or a plurality of doses over a period of time.

Whilst it is possible for a compound of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

Proteins and peptides may be delivered using an injectable sustained-release drug delivery system. These are designed specifically to reduce the frequency of injections. An example of such a system is Nutropin Depot which encapsulates recombinant human growth hormone (rhGH) in biodegradable microspheres that, once injected, release rhGH slowly over a sustained period.

The protein and peptide can be administered by a surgically implanted device that releases the drug directly to the required site. For example, Vitrasert releases ganciclovir directly into the eye to treat CMV retinitis. The direct application of this toxic agent to the site of disease achieves effective therapy without the drug's significant systemic side-effects.

Electroporation therapy (EPT) systems can also be employed for the administration of proteins and peptides. A device which delivers a pulsed electric field to cells increases the permeability of the cell membranes to the drug, resulting in a significant enhancement of intracellular drug delivery.

Proteins and peptides can be delivered by electroincorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of protein and peptide delivery is the ReGel injectable system that is thermo-sensitive. Below body temperature, ReGel is an injectable liquid while at body temperature it immediately forms a gel reservoir that slowly erodes and dissolves into known, safe, biodegradable polymers. The active drug is delivered over time as the biopolymers dissolve.

Protein and peptide pharmaceuticals can also be delivered orally. The process employs a natural process for oral uptake of vitamin B₁₂ in the body to co-deliver proteins and peptides. By riding the vitamin B₁₂ uptake system, the protein or peptide can move through the intestinal wall. Complexes are synthesised between vitamin B₁₂ analogues and the drug that retain both significant affinity for intrinsic factor (IF) in the vitamin B₁₂ portion of the complex and significant bioactivity of the drug portion of the complex.

Proteins and polypeptides can be introduced to cells by "Trojan peptides". These are a class of polypeptides called penetratins which have translocating properties and are capable of carrying hydrophilic compounds across the plasma membrane. This system allows direct targetting of oligopeptides to the cytoplasm and nucleus, and may be non-cell type specific and highly efficient. See *Derossi et al* (1998), *Trends Cell Biol* **8,** 84-87.

As used herein "pharmceutically effective amount" includes within its meaning the optimum amount of compound, agent or inhibitor of the invention to be administered to the individual. This can be determined by a person skilled in the art taking into account the species, size, age and health of the patient or subject. The optimum amount will be the amount that is able to best inhibit the activity of a Tec family PTKs without causing unacceptable levels unwanted side effects, such as cytotoxic effects or immune responses. The skilled person will be able to determine whether a dose has an unacceptable side effect. The amount of compound, agent or inhibitor to be administered in a single dose is typically from 1 ng to 100g, more typically from 1µg to 10g, yet more typically from 1mg to 1g, preferably from 10mg to 100 mg. As a general proposition, the total pharmaceutically effective amount of inhibitor, preferably administered parenterally, per dose will be in the range of 1 µg/kg/day to 10 mg/kg/day of a patient body-weight. More preferably this dose is at least 0.01 mg/kg/day, most preferably in humans between about 0.01 and I mg/kg/day.

Inhibitors use by and identified by methods of the invention may be used to study the inhibition of sepsis, and/or septic shock caused by stimuli such as but not limitted to TRR ligands, LPS and zymosan. Typically the study is *in vitro*.

The invention will now be described in more detail by reference to the following Figures and Examples wherein:
**Figure 1.** Btk is activated in response to LPS stimulation of RAW 264.7 murine macrophages and primary human monocytes.
**Figure 2.** Overexpression of Btk but not PyK-2 increase LPS induced TNF expression in PBMCs.
**Figure 3.** Effect of a specific Btk inhibitor on LPS-induced TNF-α production in RAW 264.7 murine macrophages.
**Figure 4.** Stimulus-induced cytokine expression in PBMCs from XLA and normal donors. Figures 4 (a), (b) & (e) LPS-induced TNFα expression in PBMCs from XLA and normal donors. Figure 4 (d) LPS-induced IL-1β expression in PBMCs from XLA and normal donors. Figure 4 (e) zymosan-induced TNFα expression in PBMCs from XLA and normal donors.
**Figure 5.** Pathways controlling IκBα degradation induced by LPS do not appear to require tyrosine kinase.
**Figure 6.** LPS can still induce IκBα degradation in XLA PBMC.
**Figure 7.** Maturing human XLA monocytes with M-CSF restores TNF production.
**Figure 8**. Maturing monocytes with M-CSF up-regulates Tec expression.
**Figure 9.** LPS activates Tec kinase in human M-CSF monocytes.
**Figure 10**. Tyrosine kinase inhibitor herbimycin blocks spontanteous TNF production in RA synovial membrane cultures.
**Figure 11.** The nucleotide sequence of Btk. The sequence has the following features: misc_feature 173..562 /note="PH; Region: PH domain"; misc_feature 566..673 /note="Btk; Region: Btk motif"; misc_feature 566..673 /note="Btk; Region: Bruton's tyrosine kinase Cys-rich motif"; misc_feature 812..970 /note="SH3; Region: Src homology 3 domains"; misc_feature 812..976 /note="SH3; Region: SH3 domain"; misc_feature 998..1267 /note="SH2; Region: Src homology 2 domains"; misc_feature 1004..1249 /note="SH2; Region: Src homology domain 2"; misc_feature 1367..2053 /note="pkinase; Region: Eukaryotic protein kinase domain"; misc_feature 1367..2065 /note="TyrKc; Region: Tyrosine kinase, catalytic domain"; misc_feature 1373..2056 /note="S_TKc; Region: Serine/Threonine protein kinases, catalytic domain"
**Figure 12.** The polypeptide sequence of Btk.
**Figure 13.** The nucleotide sequence of Tec. The sequence has the following features: misc_feature 72..365 /note="PH; Region: PH domain"; misc_feature 414..497 /note="Btk; Region: Btk motif"; misc_feature 414..497 /note="Btk; Region: Bruton's tyrosine kinase Cys-rich motif'; misc_feature 600..758 /note="SH3; Region: Src homology 3 domains"; misc_feature 600..761 /note="SH3; Region: SH3 domain"; misc_feature 789..1064 /note="SH2; Region: Src homology 2 domains"; misc_feature 795..1046 /note="SH2; Region: Src homology domain 2"; misc_feature 1164..1904 /note="pkinase; Region: Eukaryotic protein kinase domain"; misc_feature 1164..1907 /note="TyrKc; Region: Tyrosine kinase, catalytic domain"; misc_feature 1170..1754 /note="S_TKc; Region: Serine/Threonine protein kinases, catalytic domain"
**Figure 14**. The polypeptide sequence of Tec.
**Figure 15.** The nucleotide sequence of Itk. The sequence has the following features: misc_feature 206..313/note="Btk; Region: Bruton's tyrosine kinase Cys-rich motif"; misc_feature 206..313/note="Btk; Region: Btk motif"; misc_feature 389..547/note="SH3; Region: SH3 domain"; misc_feature 395..544/note="SH3; Region: Src homology 3 domains"; misc_feature 578..856/note="SH2; Region: Src homology 2 domains"; misc_feature 584..838/note="SH2; Region: Src homology domain 2"; misc_feature 956..1705/note="TyrKc; Region: Tyrosine kinase, catalytic domain"; misc_feature 956..1705/note="pkinase; Region: Eukaryotic protein kinase domain"; misc_feature 962..1591/note="S_TKc; Region: Serine/Threonine protein kinases, catalytic domain"
**Figure 16.** The polypeptide sequence of Itk.
**Figure 17**. The polypeptide sequence of Txk. The sequence has the following features: Protein 1..531/product="TXK tyrosine kinase; Region 86..137/region_name="Src homology 3 domains"/db_xref ="CDD:SH3/note ="SH3"; Region 86..139/region_name="SH3 domain" /db_xref= "CDD:pfam00018"/note="SH3"; Region 148..237/region_ name="Src homology 2 domains"/db_xref="CDD:SH2" /note="SH2"; Region 150..231 /region_name="Src homology domain 2" /db_xref="CDD:pfam00017" /note="SH2"; Region 271..522 /region_name="Tyrosine kinase, catalytic domain" /db_xref="CDD:TyrKc /note="TyrKc"; Region 273..504/region_name="Serine/Threonine protein kinases, catalytic domain" /db_xref="CDD:S_TKc" /note="S_TKc"; Region 273..521 /region_name= "Eukaryotic protein kinase domain" /db_xref= "CDD: pfam00069" / note="pkinase
**Figure 18.** The nucleotide sequence of Bmx. The sequence has the following features: misc_feature 49..366 /note="PH; Region: PH domain"; misc_feature 370..477 /note="Btk; Region: Btk motif"; misc_feature 370..477 /note="Btk; Region: Bruton's tyrosine kinase Cys-rich motif"; misc_feature 913..1182 /note="SH2; Region: Src homology 2 domains" misc_feature 919..1164 /note="SH2; Region: Src homology domain 2"; misc_feature 1282..2031 /note="TyrKc; Region: Tyrosine kinase, catalytic domain"; misc_feature 1282..2022 /note="pkinase; Region: Eukaryotic protein kinase domain"; misc_feature 1288..2049 /note="S_TKc; Region: Serine/Threonine protein kinases,catalytic domain"
**Figure 19.** The polypeptide sequence of Bmx.

### EXAMPLE

### METHODS

### Isolation and culture of human monocytes

Human peripheral blood monocytes were freshly prepared from the buffy coat fraction of a unit of blood from a single donor. Mononuclear cells were prepared by Ficoll-Hypaque centrifugation on a Lymphoprep gradient and monocytes were isolated by centrifugal elutriation on a Beckman JE6 elutriator (High Wycombe, United Kingdom) using RPMI 1640 medium with 2 mM L-glutamine and 1% heat-inactivated foetal calf serum. Monocyte purity was routinely assessed by flourescence-activated cell-sorting forward/side scatter measurements in a Becton Dickinson FACScan. Monocyte fractions of >85% purity were routinely collected in this manner. Monocytes were cultured in RPMI 1640 medium with 2 mM L-glutamine, 100 units/ml penicillin/streptomycin and 10% heat-inactivated fetal calf serum at 37°C in a humidified atmosphere containing 5% CO₂. Monocytes were treated with M-CSF (100 ng/ml), (generous gift from Glenn Larsen, Genetics Institute, Boston) for 48 h prior to viral infection.

### Culture of murine macrophage cell line, Raw 264.7

The murine macrophage cell line, Raw 264.7 were maintained in DMEM medium supplemented with 100 units/ml penicillin/streptomycin and 5% heat-inactivated fetal calf serum at 37°C in a humidified atmosphere containing 5% CO₂.

### Rheumatoid synovial cell preparation

Synovium from rheumatoid patients undergoing joint replacement surgery was dissociated by cutting into small pieces, digested with collagenase and DNase (Buchan et al., 1988). The total cell mixture consisting predominantly of T cells and macrophages were cultured at a density of 1 X 10⁶ cells/ml in RPMI containing 100 units/ml penicillin/streptomycin and 5% heat-inactivated fetal calf serum.

### Isolation and culture of PBMCs from XLA and control donors

Human blood samples were collected into Lithium heparin vacutainers. Each blood sample was mixed with an equal volume of Hank's Balanced Salt Solution (HBSS). Peripheral blood mononuclear cells (PBMCs) were prepared by Ficoll-Hypaque centrifugation on a Lymphoprep gradient. PBMCs were resuspended in RPMI containing 100 units/ml penicillin/streptomycin and 10% heat-inactivated foetal calf serum and cultured as described for monocytes above. In some cases monocytes were isolated from the PBMCs by adherance to plastic for 1h at 37°C in RPMI/10% FCS. Non-adherant cells were subsequently washed off and the adherant macrophages were rested overnight and then stimulated with LPS. Control blood was taken from healthy males of a similar age range to the XLA patient.

### Adenoviral vectors and their propagation

The pAdEasy-1 adenoviral plasmid was provided by B. Vogelstein (The Howard Hughes Medical Institute, Baltimore, MD). A plasmid containing human wild type Btk cDNA was provided by Dr. Christine Kinnon (Institute of Child Health, London, U.K.). An adenovirus was constructed encoding wild type human Btk using the AdEasy system as described by He *et al*. (1998, *Proc. Natl. Acad. Sci. USA,* **95,** 2509-2514). Recombinant viruses were generated in BJ5183 bacterial cells transformed by the heat-shock method with 1 µg of linearised modified AdTrack constructs and 100ng of replication-deficient adenoviral vector, pAdEasy-1. Positive recombinant clones were selected through their resistance to kanamycin. Following selection, DNA was extracted and used for virus propagation in 293 human embryonic kidney cells. Viruses were purified by ultracentrifugation through two cesium chloride gradients as described by He *et al*. (1998, *Proc. Natl. Acad. Sci. USA,* **95,** 2509-2514). Titres of viral stocks were determined by plaque assay in 293 cells after exposure for 1 hour in serum free DMEM medium (Gibco BRL) and subsequently overlayed with agarose/DMEM and incubated for 10-14 days.

Each adenovirus encodes a version of the Btk gene under the control of a cytomegalovirus (CMV) promoter and also the gene for green fluorescent protein (GFP) under a CMV promoter. The GFP enabled infection efficiencies in cells to be assessed using a UV light microscope. Recombinant, replication deficient adenoviral vectors encoding GFP (AdGFP) were kindly provided by Dr. Cathleen Ciesielski and Dr. Clive Smith (Kennedy Institute, London, United Kingdom).

The GFP (AdGFP) was made as above using the Vogelstein reagents using the method of He *et al*.. (1998, *Proc. Natl. Acad. Sci. USA*, **95,** 2509-2514) using techniques known in the art.

### Viral Infection

Macrophages derived from monocytes following M-CSF (100 ng/ml) treatment for 48 h were washed in serum-free RPMI medium and then exposed to virus at different multiplicity of infection (m.o.i.) for 1 hour in serum-free RPMI at 37°C. Following exposure to virus, macrophages were washed in RPMI and cultured in complete medium for 24 h or 48 h. Macrophages were then either assessed for expression of Btk by lysis and Western blotting or stimulated with LPS for various time periods.

### Immunoprecipitation and in vitro kinase assay

Btk autokinase activity was measured in response to LPS in Raw 246.7 murine macrophages and primary human monocytes. Raw 246.7 cells were rested overnight either in 0.5% FCS/DMEM, or in serum-free DMEM, preferably in in serum-free DMEM.

Cells were then stimulated with LPS (10ng/ml) for various times and then pelleted quickly. Pellets were lysed in 1% NP40 lysis buffer containing 20 mM Tris pH 8, 130 mM NaCl, 10 mM NaF, 1 mM DTT, 20 µM leupeptin or 10 µM E64, 100 µM sodium orthovanadate, 1 mM PMSF and 2 mg/ml aprotinin. Cells were lysed for 20 min on ice and then centrifuged in a microfuge for 10 min at 1300rpm at 4°C. The supernatants were removed and precleared for 30 min at 4°C in 30 µl protein A (previously washed in lysis buffer).

With cells prepared according to protocol 1, after centrifuging the samples for 10 min at 1300rpm, the supernatants were removed and incubated with 4 µl of polyclonal anti-Btk M-138 (Santa Cruz) or polyclonal rabbit anti-Btk (gift from C. Kinnon, Institute of Child Health, London) for 1 h at 4°C. Protein A (20 µl) was then added to each sample and incubated for 1.5 h at 4°C. Beads were then pelleted and washed 4X in lysis buffer, and 1X in kinase buffer (10 mM MgCl₂; 10 mM MnCl₂). Washes for polyclonal anti-Btk M-138 (Santa Cruz) further included 1x RIPA buffer. Beads were mixed with 30 µl kinase buffer containing _{γ}³²P [ATP] and incubated for 15 min at room temperature. The reaction was stopped by the addition of 2X Laemilli buffer gel sample buffer and the samples were boiled for 3 min.

Samples were electrophoresed on an 8% SDS polyacrylamide gel. The gel was stained, fixed, dried and autoradiographed.

Tec autokinase activity was measured as described previously by Mano *et al* (1995, *Blood,* **82,** 343-350). Monocytes were incubated with M-CSF (100ng/ml) for 48h and then rested overnight without M-CSF before stimulation with LPS. Tec protein was immunoprecipitated either with rabbit anti-Tec serum (gift from Dr. Mano, Jichi Medical School, Toshigi, Japan) or goat polyclonal anti-Tec (Santa Cruz).

### Westerns blots

Cell lysates were prepared as outlined above using lysis buffer containing 1% Trition X100, 20mM Hepes, pH 7.4, 50mM β-glycerophosphate, 2mM EGTA, 150mM NaCl, 10% glycerine, 1mM DTT, 1mM Na orthovanadate, 1mM PMSF, 10mM NaF, 1µg/ml pepstatin, 3µg/ml aprotinin 10µM E64. Lysates were electrophoresed on a 8% or 10% SDS-polyacrylamide gel and transferred onto PVDF membrane. The membranes were blocked in 5% Marvel/TSB-Tween 20 (0.1%) containing 10mM NaF and 1mM Na orthovanadate for 1h at room temperature. After blocking, membranes were probed either with 1µg/ml anti-Btk (Pharminogen) or 1:1000 anti-IκBα (Santa Cruz) or 1:1000 anti- p54 (JNK) (kindly provided by Professor Saklatvala, Imperial College, London) in 5% marvel/PBS/Tween 20 (0.05%). Tec protein was detected using either rabbit anti-Tec serum (gift from Dr. Mano, Jichi Medical School, Toshigi, Japan) or goat polyclonal anti-Tec (Santa Cruz). HRP-conjugated anti-rabbit or anti-goat immunoglobulins (Dako) diluted 1:2000 in 5% Marvel/TBS-Tween-20 (0.1%) were used as secondary antibody. Following extensive washing in TBS/Tween (0.1%) the membranes were developed using enhanced chemiluminescence (ECL) according to manufacturer's instructions.

### Enzyme-linked Immunosorbent Assay (ELISA)

Supernatants from experiments using either human macrophages, rheumatoid synovial cells or PBMCs were analyzed for TNF-α ot IL-1 by enzyme-linked immunosorbent assay (ELISA). The ELISA for hTNF-α was performed as described by Engelberts *et al*. (1991) *Lymphokine Cytokine Res.* **10,** 60-76.

### RESULTS

### Btk is activated upon LPS stimulation

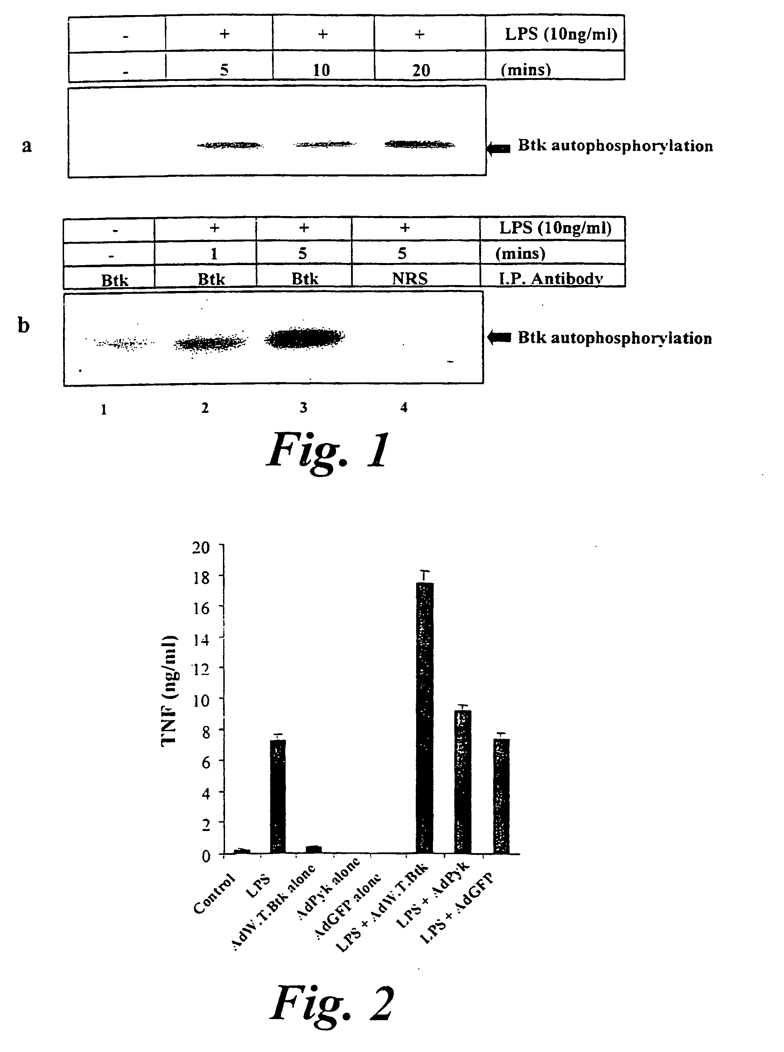

Figure 1(a) shows that LPS (10ng/ml) stimulation of the murine macrophage cell line, Raw 264.7 cells induced a 2-3 fold increase in Btk autokinase activity above basal levels. Autophosphorylation of Btk could be detected at 5 min and peaked between 20-30 min (Fig 1). Dose response studies carried out at the optimal stimulation time of 20 min showed a typical dose responsive increase in autokinase activity of Btk in response to LPS, which peaked between 10-100 ng/ml LPS (data not shown).

Figure 1(b) shows that Btk is activated in response to LPS stimulation of primary human monocytes. Primary human monocytes (B) were stimulated with LPS (10ng/ml) for the indicated intervals. Cells were lysed and immunoprecipitated with either anti-Btk antibody or non-immune rabbit serum (NRS). Immune complex kinase assays were performed and autophosphorylated Btk proteins were detected by autoradiography of the SDS-PAGE gel. This result is representative of three separate experiments.

### Overexpression of Btk adenovirus enhances LPS-induced cytokine production

Cells were infected either with AdW.T.Btk or the contol virus, Adβ-Gal (gift from Drs. A Byrne and A Wood, Oxford University) and then either left untreated or stimulated with LPS (10ng/ml) for 4h. The supernatants were harvested and assayed for TNF-α by ELISA as described above. TNF-α levels in supernatants from uninfected control cells were below the detection limit of the ELISA (<50pg/ml) (data not shown). Infection with AdW.T.Btk alone did not cause the macrophages to produce TNF-α (data not shown). Following 24h stimulation with LPS uninfected macrophages produced 3 +/- 0.4 ng/ml TNF-a (data not shown). Infection with 50:1 Adβ-Gal (control virus) did not significantly effect the response of these cells to LPS (data not shown). Infection with 25:1 AdW.T.Btk caused a 1.6 fold increase in TNF-α expression over uninfected LPS-stimulated cells (data not shown). Furthermore, infection with 50:1 AdW.T.Btk caused a 1.7 fold augmentation of TNF-α expression (data not shown).

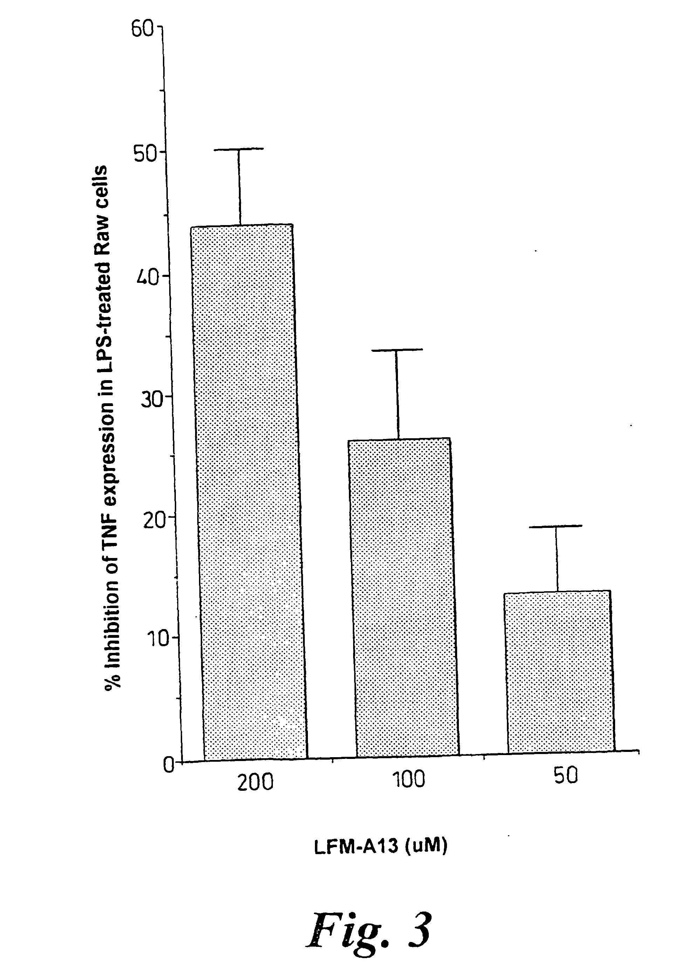

To confirm these results, M-CSF(100ng/ml) treated monocytes from normal donors were infected or uninfected with the indiciated viruses. Cells were activated by 10 ng/ml LPS where shown. After 24 h culture supernatants were harvested and analysed for TNF production by ELISA Over expression of Btk lead to an enhanced production of TNF (Figure 2). By contrast, over expression of pyk2 using the same approach produced an insignificant effect (Figure 2). The effect of a Btk mutein (defective in enzymatic activity) produced no effect on TNF production (not shown), which demonstrates that the effect of Btk is dependant on kinase activity. In the absence of LPS overexpression of Btk induces low production of TNF over background.

### Effects of a specific Btk inhibitor, LFM-A13, on LPS-induced cytokine production in Raw 264.7 cells

LFM-A13 has been shown to inhibit Btk function both *in vitro* and *in vivo* (Mahajan *et al*., 1999). This compound was investigated for its effects on TNF-α production. RAW 264.7 murine macrophages were pretreated with various concentrations of LFM-A13 or vehicle control (0.05% DMSO) for 1h prior to stimulation with LPS (10ng/ml) for 4h at 37°C. Supernatants were then harvested and assayed for TNF-α by ELISA as described in the Methods section. LFM-A13 was found to block TNF-α expression in a dose-dependent manner, giving a 44% inhibition at 200 µM (Fig. 3). The use of this compound above this concentration was not possible due to the toxicity that this compound exhibited.

### TRR ligand-induced cytokine production in PBMCs from XLA patients and control donors

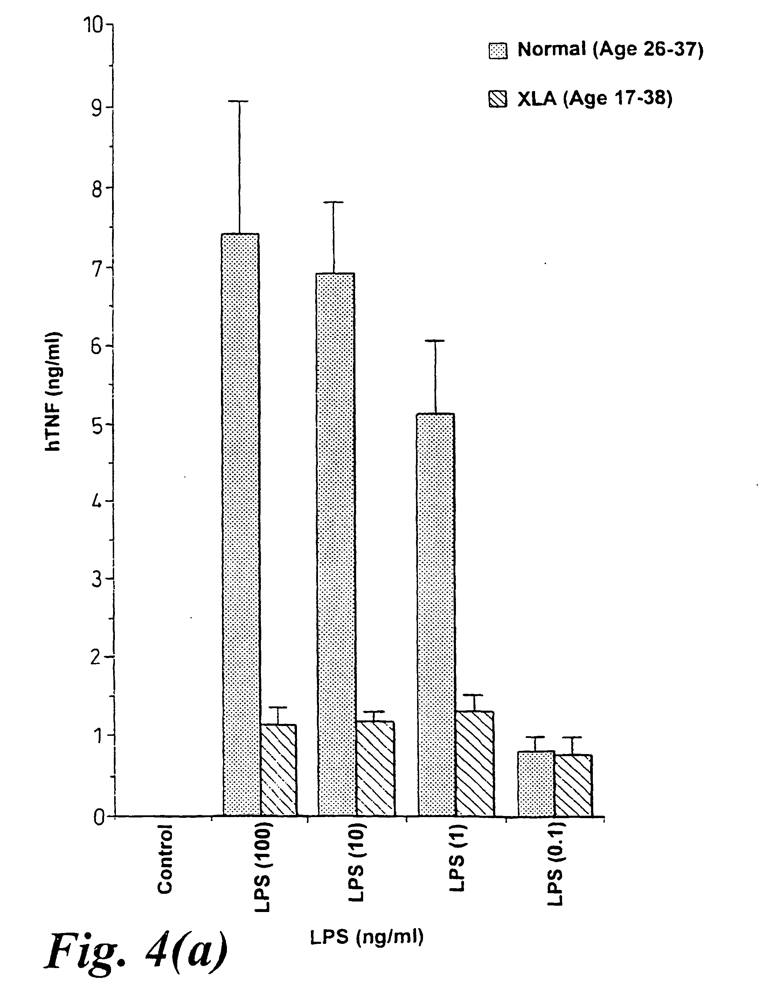

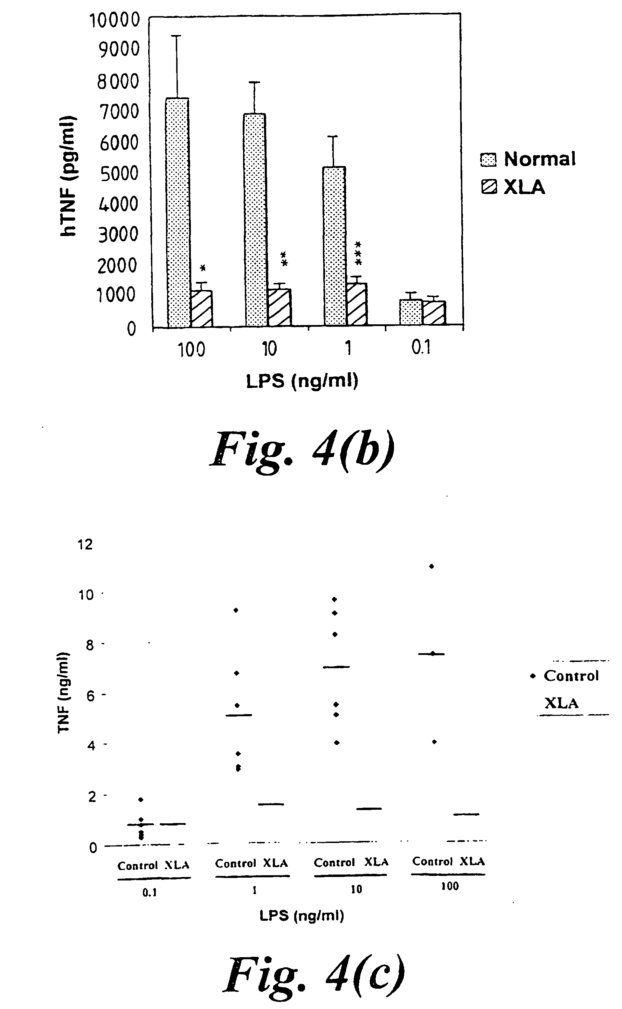

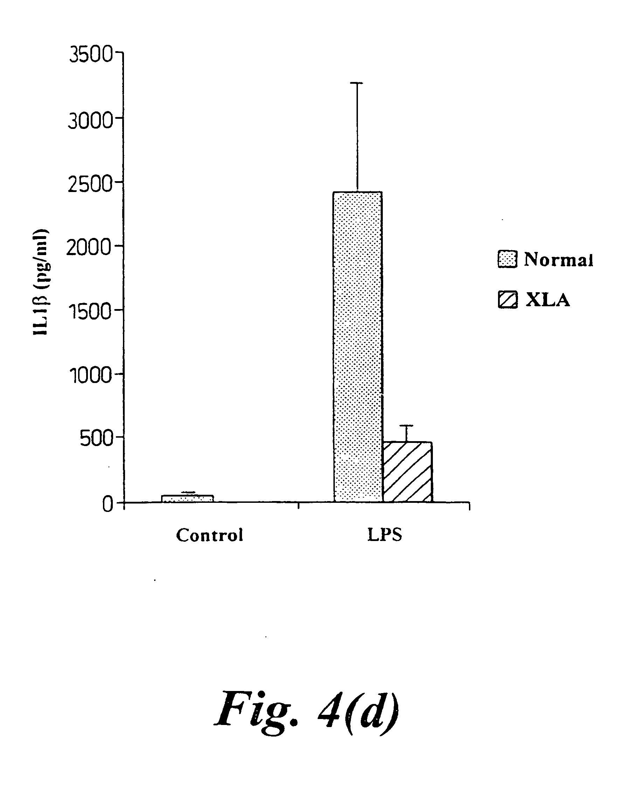

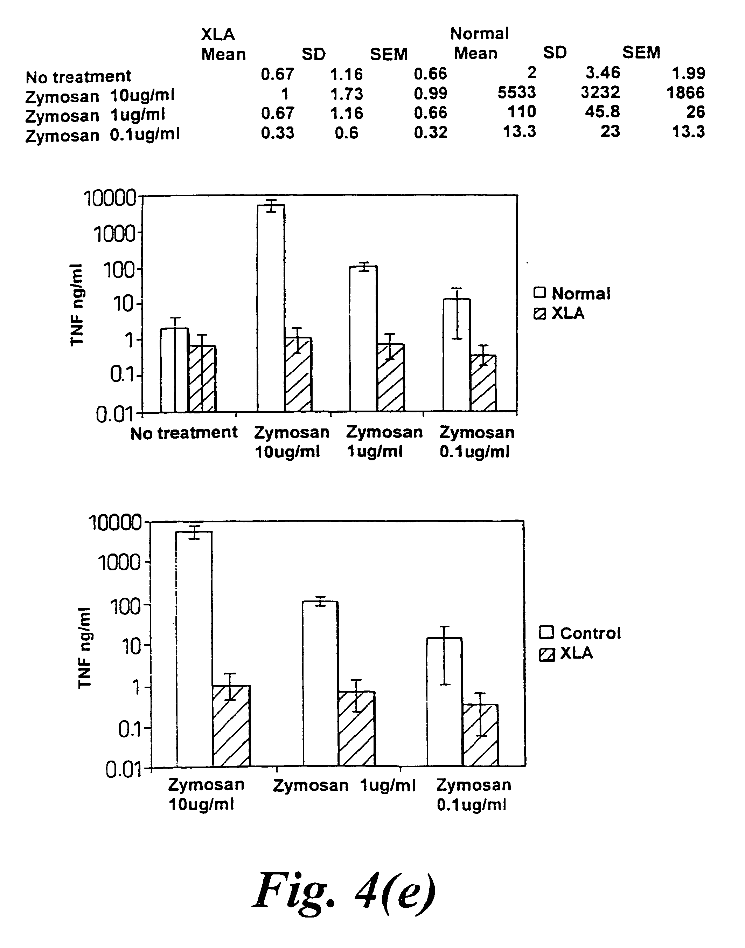

To confirm the role of Btk in controlling TNF-α production we also investigated the response of XLA patients, who are deficient in Btk expression. Figure 4(a) shows the response of PBMCs isolated from the whole blood samples of XLA and normal male donors. PBMCs were stimulated with various concentrations of LPS (0.1-100 ng/ml) for 18h. Supernatants were harvested and assayed for TNF-α by ELISA as described in the Methods section. PBMCs from XLA patients consistently expressed less TNF-α in response to LPS at concentrations between 1-100 ng/ml. This reduction in TNF expression in these patients compared to controls was statistically significant.

To confirm this result, LPS-induced TNF-α expression was assayed in PBMCs from XLA and normal donors. PBMC were isolated from whole blood samples of XLA and normal male donors. PBMCs were then stimulated with various concentrations ofLPS (0.1-100 ng/ml) for 18h. Supernatants were then harvested and assayed for TNF-α by ELISA. Student t-test P values show that the impairment of the XLA response was significant: *p<0.05, **<0.001, ***<0.01 as shown in Figure 4(b). The TNF response of XLA, PBMC to LPS is greatly impaired, with the production of cytokines being only 20% of normal (Figure 4(c)).

As with the assay for TNF-α production described above, LPS-induced IL-1β production was also impaired in XLA PBMC as shown in Figure 4(d). The impaired response of XLA PMBC was not only restricted to LPS as zymosan activation was also greatly attenuated in the Btk deficient cells as shown in Figure 4(e). As zymosan is thought to act via TLR2, this would indicate that Btk, and potentially other Tec family PTK members, may be involved in the function of Toll-like receptors generally.

### LPS can still induce IKK activation/IκBα degradation in XLA PBMC

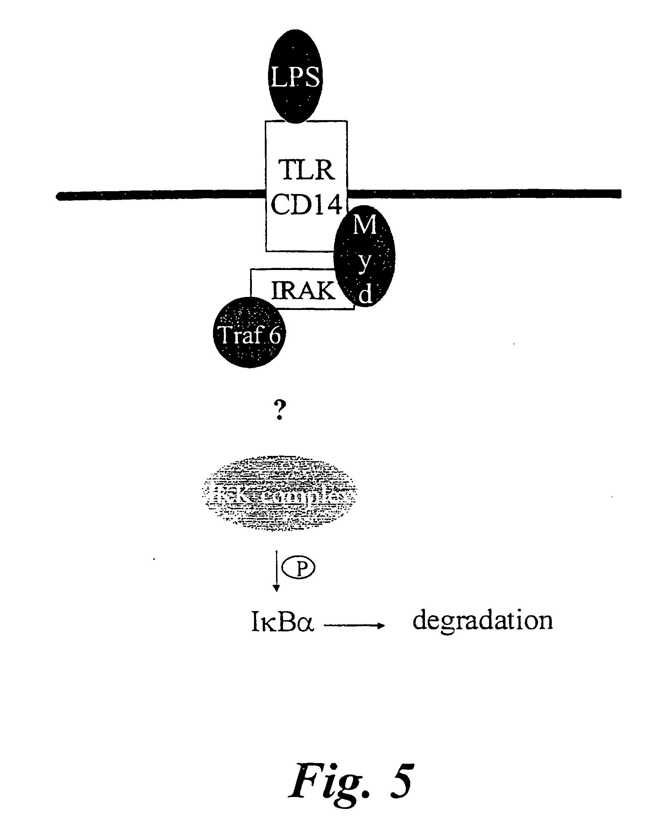

The low of response of XLA PBMC lead us to investigate whether the defect was a direct effect on the absence of Btk or a secondary effect related to impaired monocyte development. The expression of the LPS receptor CD 14 was normal on XLA monocytes (results not shown) and previous investigations of XLA patients shown normal levels of circulating monocytes. We therefore investigated the function of internal signalling pathways. LPS activation of the IκBα kinases (IKK) is thought to be independent of tyrosine kinase activity (Figure 5).

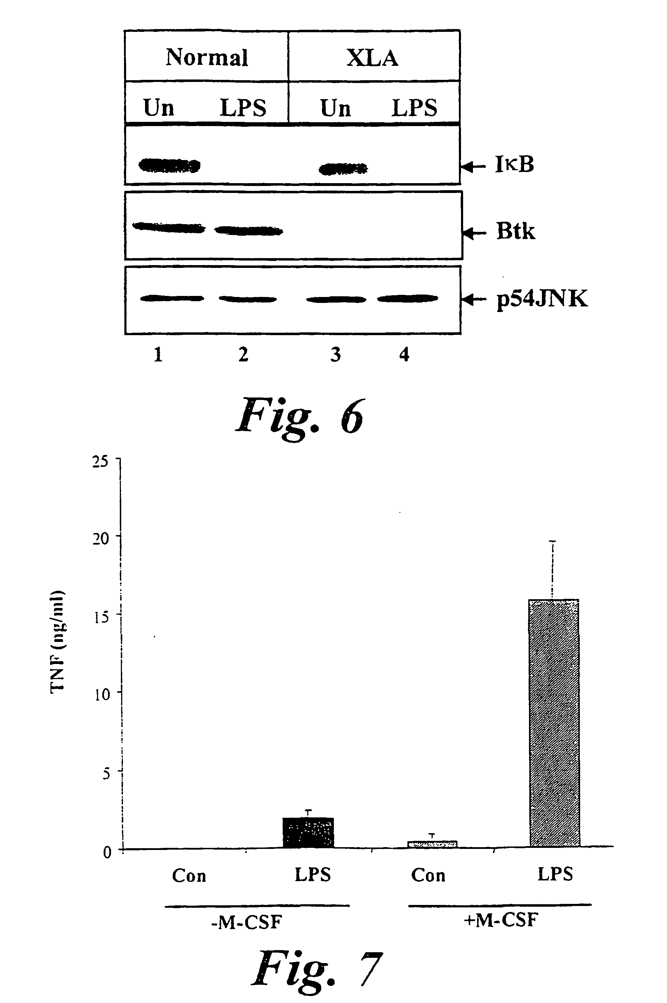

Monocytes were isolated from PBMCs by plastic adherence. Monocytes were stimulated with LPS (10ng/ml) for 20 minutes. Cells were pelleted and lysed as described in "Methods". Proteins were separated on 8% SDS-polyacrylamide gel and transferred onto nitrocellulose. Blots were probed with antibodies to IκBα or Btk. Blots were subsequently stripped and re-probed for the non-phosphorylated form of p54Jnk to show equal loading of gels. Certain studies have shown that the activation of NFκB DNA binding activity by LPS is resistant to tyrosine kinase activity (Delude *et al*. (1994) *J Biol Chem*., **269,** 22253; Yoza *et al.* (1996) *J Biol Chem.,* **271,** 18306). In agreement with this previous data we observed that IκBα degradation induced by LPS was normal in XLA PBMC (Figure 6). These data would indicate that the impaired TNF production is directly related to absence of Btk and not a more general down regulation of all LPS responses.

### Maturing human XLA monocytes with M-CSF restores TNF production

The above data with XLA derived cells and the production of TNF was a surprise, as XLA patients do not shown any obvious impairment of their innate immune response. Moreover previous studies on macrophage or splenocytes have generally concluded that the production of cytokines is normal, although NO production has been shown to be impaired (Hata *et al.* (1998) *J. Exp. Med.,* **187**, 1235; Mukhopadhyay *et al*. (1999) *J. Immunol*., **163,** 1786). However it is possible that the role of Btk is different in mice and humans especially as the phenotype of XLA and *xid* is not identical. An alternative explanation was that our studies have measured the response of monocytes whereas the studies on mice used matured macrophages and thus there is the possibility that there is a different requirement for Btk during myleoid differentiation.

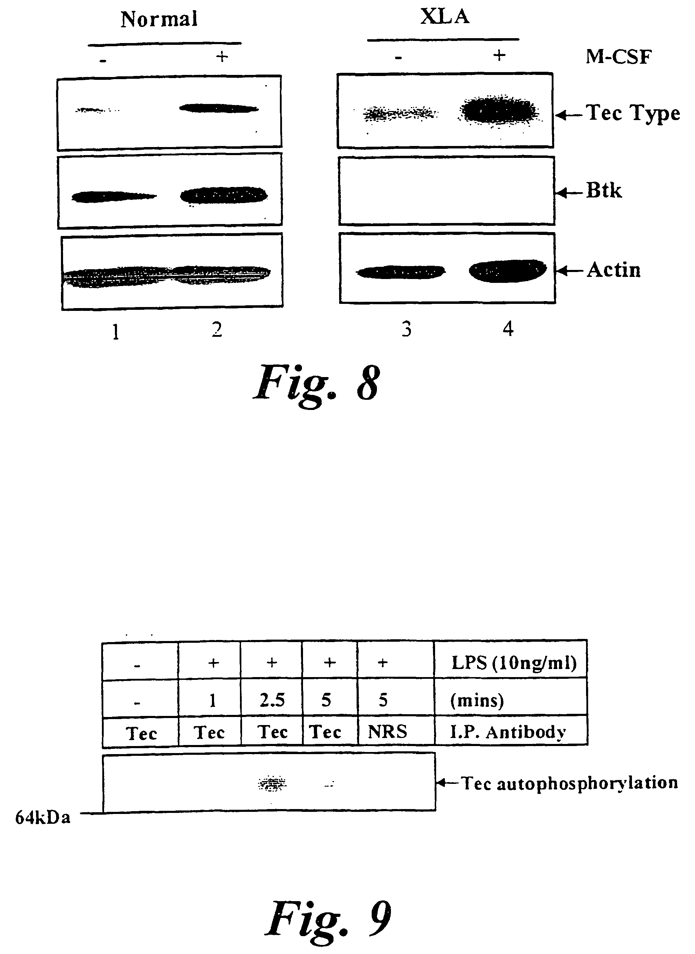

The effect of M-CSF treatment on LPS-stimulated TNFα production in XLA monocytes was assayed. Monocytes were purified by plastic adherence from PBMCs using blood from XLA or normal donors. Cell were either stimulated with LPS (10ng/ml) for 2h immediately or incubated with M-CSF (100ng/ml) for 48h prior to LPS stimulation. Supernatants were harvested and assayed for TNFα expression by ELISA. Maturation of XLA monocytes along the macrophage lineage with M-CSF greatly improved the production of TNF in response to LPS (Figure 7) indicating that there was a decreased requirement for Btk. These data therefore support the above hypothesis that there is the possibility that there is a different requirement for Btk during myleoid differentiation.

### Maturing monocytes up-regulate Tec expression

Together these data suggested that macrophages lost their absolute requirement for Btk. We investigated the reasons for this, in particular, the possibility that another member of the Tec family of tyrosine kinase, Tec itself, a kinase also reported to be expressed in monocytic cells, may be compensating for Btk. The compensation of function between the members of the same tyrosine kinases family is known.

The effect of M-CSF treatment on Tec expression in primary human monocytes was assayed. Monocytes were isolated from PBMCs by plastic adherence using blood from either normal (Figure 8, lanes 1&2) or XLA donors (Figure 8, lanes 3&4). Monocytes were then either lysed immediately or incubated with M-CSF (100ng/ml) for 48 hours and then lysed. Cells lysates were prepared and proteins were separated on 7.5% SDS-polyacrylamide gel and transferred onto nitrocellulose. Blots were probed either with anti-TecSH3, anti-Btk (Santa Cruz) or actin antibody. We observed that expression of Tec was low in purified monocytes from normal or XLA blood, however activation of the monocytes by M-CSF caused a great up-regulation in the expression of Tec kinase (Figure 8).

### LPS activates Tec kinase in Human M-CSF monocytes

For Tec to compensate for Btk it should be activated by LPS, this was demonstrated by the data in figure 9. Monocytes were isolated from PBMCs by centrifugal elutriation using blood from normal donors. Monocytes were incubated with M-CSF (100ng/ml) for 48h and then stimulated with LPS for the indicated time periods. Tec was immunoprecipitated using anti-Tec (Santa-Cruz) antibody or an irrelevant antibody (NRS) *and in vitro* autokinase assay was performed as described in "Methods". Immunocomplexes were separated on 7.5% SDS-polyacrylamide gel. The gel was stained with Coomassie Blue, dried and was subjected to autoradiography. Figure 9 shows a rapid and potent activation of Tec. These data demonstrate that Tec tyrosine kinases are potentially important modulators of TNF expression and this is the first tyrosine kinase whose activation has been linked to the expression of TNF.

### Inhibition of tyrosine kinase activity blocks spontaneous TNF production in rheumatoid arthritis synovial membrane culture

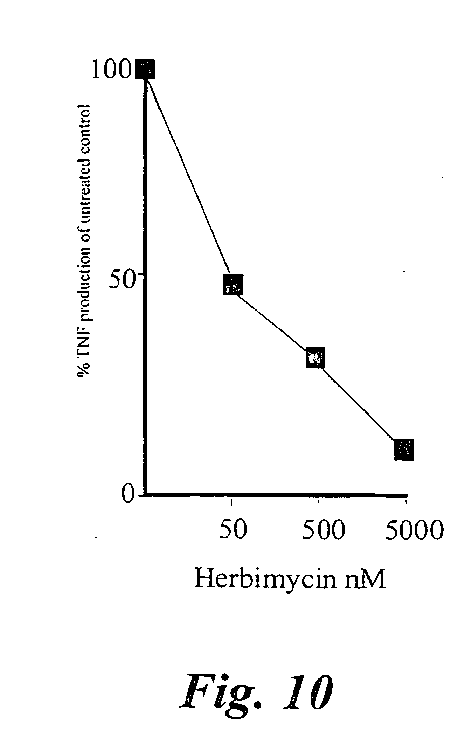

RA synovial cultures were treated with herbimycin at the given concentrations. Following overnight culture the supernatants were harvested and TNF expression measured by ELISA. We have demonstrated that herbimycin is a potent inhibitor of TNF production from RA synovial membrane cultures. This indicates that tyrosine kinase activity is also required by the stimulus driving TNF production in RA (Figure 10).

## Claims

1. Use of an inhibitor of a member or members of the Tec family of PTKs in the manufacture of a medicament for use in the treatment of a condition associated with cytokine production, wherein said condition is rheumatoid arthritis or a condition resulting from an infection.

2. A use according to claim 1 wherein the condition is a tumour necrosis factor (TNF) associated condition.

3. A use according to claim 2 wherein the TNF is TNFα.

4. A use according to claim 1 wherein the condition is an interleukin-1 (IL-1) associated condition.

5. A use according to claim 4 wherein the IL-1 is IL-1β.

6. A use according to any one of the preceding claims wherein the condition is sepsis.

7. A use according to any one of claims 1 to 5 wherein the condition is septic shock.

8. A use according to any one of the preceding claims wherein the condition is induced by a Toll Related Receptor (TRR) ligand.

9. A use according to any one of the preceding claims wherein the condition is induced by lipopolysaccharide (LPS).

10. A use according to any one of the preceding claims wherein the condition is induced by zymosan.

11. A use according to any one of claims 1 to 9 wherein the condition is induced by Gram-negative bacteria.

12. A use according to any one of the preceding claims wherein the inhibitor is specific for a member or members of the Tec family ofPTKs.

13. A use according to any one of the preceding claims wherein the member of the Tec family of PTKs is Bruton's tyrosine kinase (Btk).

14. A use according to any one of claims 1 to 12 wherein the member of the Tec family of PTKs is Tec.

15. A use according to any one of the preceding claims wherein the inhibitor is a chemical inhibitor.

16. A use according to claim 15 wherein the inhibitor is LFM-A13.

17. A use according to any one of claims 1 to 14, wherein the inhibitor is an antibody or a fragment thereof which is capable of binding specifically to a member or members of the Tec family of PTKs or a fragment thereof.

18. A use according to any one of claims 1 to 14, wherein the inhibitor is a nucleic acid capable of reducing the expression of a member or members of the Tec family of PTKs.

19. A use according to claim 18 wherein the nucleic acid comprises the sequence or complementary sequence as defined in any one of Figure 11, 13, 15 or 18, or the sequence of a nucleic acid that encodes a polypeptide as defined in any one of Figures 12, 14, 16, 17 or 19, or a complementary sequence thereof, a variant of any one of these sequences, or a fragment of any of the above.

20. A method for identifying an inhibitor of a member or members of the Tec family ofPTKs which inhibitor is suitable for use in the treatment of a condition associated with cytokine production wherein said condition is rheumatoid arthritis or a condition resulting from an infection, the method comprising:
(a) providing, as a first component, a cell capable of TRR ligand-induced cytokine production;
(b) providing, as a second component, a TRR ligand;
(c) contacting the first and second components in the presence of a test agent;
(d) determining whether the test agent is able to inhibit the TRR ligand-induced activity of a member of the Tec family of PTKs;
thereby to determine whether the test agent could act as an inhibitor of cytokine production.

21. A method according to claim 20 further comprising the step of determining whether the inhibitor is specific for Tec family PTKs.

22. Use of an inhibitor of a member or members of the Tec family of PTKs to study the inhibition of sepsis and/or septic shock caused by a TRR ligand, *in vitro.*

## Patentansprüche

1. Verwendung eines Inhibitors eines Mitglieds oder Mitgliedern der Tec-Familie von PTKs zur Herstellung eines Medikaments für die Verwendung bei der Behandlung eines Zustands, der mit einer Cytokinproduktion assoziiert ist, wobei der Zustand rheumatoide Arthritis ist oder ein Zustand, der sich aus einer Infektion ergibt.

2. Verwendung gemäss Anspruch 1, wobei der Zustand ein mit dem Tumornekrosefaktor (TNF) assoziierter Zustand ist.

3. Verwendung gemäss Anspruch 2, wobei der TNF TNFα ist.

4. Verwendung gemäss Anspruch 1, wobei der Zustand ein mit Interleukin-1 (IL-1) assoziierter Zustand ist.

5. Verwendung gemäss Anspruch 4, wobei das IL-1 IL-1β ist.

6. Verwendung gemäss einem der vorstehenden Ansprüche, wobei der Zustand eine Sepsis ist.

7. Verwendung gemäss einem der Ansprüche 1 bis 5, wobei der Zustand ein septischer Schock ist.

8. Verwendung gemäss einem der vorstehenden Ansprüche, wobei der Zustand durch einen den Toll-Rezeptor betreffenden (TRR)-Liganden induziert wird.

9. Verwendung gemäss einem der vorstehenden Ansprüche, wobei der Zustand durch Lipopolysaccharid (LPS) induziert wird.

10. Verwendung gemäss einem der vorstehenden Ansprüche, wobei der Zustand durch Zymosan induziert wird.

11. Verwendung gemäss einem der Ansprüche 1 bis 9, wobei der Zustand durch gram-negative Bakterien induziert wird.

12. Verwendung gemäss einem der vorstehenden Ansprüche, wobei der Inhibitor für ein Mitglied oder Mitglieder der Tec-Familie von PTKs spezifisch ist.

13. Verwendung gemäss einem der vorstehenden Ansprüche, wobei das Mitglied der Tec-Familie von PTKs Bruton-Tyrosinkinase (Btk) ist.

14. Verwendung gemäss einem der Ansprüche 1 bis 12, wobei das Mitglied der Tec-Familie von PTKs Tec ist.

15. Verwendung gemäss einem der vorstehenden Ansprüche, wobei der Inhibitor ein chemischer Inhibitor ist.

16. Verwendung gemäss Anspruch 15, wobei der Inhibitor LFM-A13 ist.

17. Verwendung gemäss einem der Ansprüche 1 bis 14, wobei der Inhibitor ein Antikörper oder ein Fragment davon ist, der/das in der Lage ist, spezifisch an ein Mitglied oder Mitglieder der Tec-Familie von PTKs oder ein Fragment davon zu binden.

18. Verwendung gemäss einem der Ansprüche 1 bis 14, wobei der Inhibitor eine Nukleinsäure ist, die in der Lage ist, die Expression eines Mitglieds oder von Mitgliedern der Tec-Familie von PTKs zu reduzieren.

19. Verwendung gemäss Anspruch 18, wobei die Nukleinsäure die Sequenz oder eine komplementäre Sequenz umfasst, wie in einer der Fig. 11, 13, 15 oder 18 definiert, oder die Sequenz einer Nukleinsäure, die ein Polypeptid codiert, wie in einer der Fig. 12, 14, 16, 17 oder 19 definiert, oder eine komplementäre Sequenz davon, eine Variante von einer dieser Sequenzen oder ein Fragment von einer der obigen.

20. Verfahren zur Identifizierung eines Inhibitors eines Mitglieds oder Mitgliedern der Tec-Familie von PTKs, wobei der Inhibitor für die Verwendung bei einer Behandlung eines Zustands geeignet ist, der mit einer Cytokinproduktion assoziiert ist, wobei der Zustand rheumatoide Arthritis ist oder ein Zustand, der sich aus einer Infektion ergibt, wobei das Verfahren folgendes umfasst:
(a) Bereitstellung einer Zelle, die zu einer TRR-Liganden-induzierten Cytokinproduktion in der Lage ist, als erste Komponente;
(b) Bereitstellung eines TRR-Liganden als zweite Komponente;
(c) Kontaktieren der ersten und zweiten Komponenten in Gegenwart eines Testmittels;
(d) Bestimmung, ob das Testmittel in der Lage ist, die TRR-Liganden-induzierte Aktivität eines Mitglieds der Tec-Familie von PTKs zu inhibieren;
wodurch bestimmt wird, ob das Testmittel als ein Inhibitor der Cytokinproduktion wirken könnte.

21. Verfahren gemäss Anspruch 20, weiterhin umfassend den Schritt der Bestimmung, ob der Inhibitor für die Tec-Familie von PTKs spezifisch ist.

22. Verwendung eines Inhibitors eines Mitglieds oder Mitgliedern der Tec-Familie von PTKs zum Studium der Inhibierung der Sepsis und/oder des septischen Schocks, ausgelöst durch einen TRR-Liganden, in vitro.

## Revendications

1. Utilisation d'un inhibiteur d'un membre ou de membres de la famille Tec des PTK dans la fabrication d'un médicament destiné à être utilisé dans le traitement d'un état associé à une production de cytokines, ledit état étant la polyarthrite rhumatoïde ou un état résultant d'une infection.

2. Utilisation selon la revendication 1, dans laquelle l'état est un état associé à un facteur nécrosant des tumeurs (TNF).

3. Utilisation selon la revendication 2, dans laquelle le TNF est TNFα.

4. Utilisation selon la revendication 1, dans laquelle l'état est un état associé à une interleukine 1 (IL-1).

5. Utilisation selon la revendication 4, dans laquelle l'IL-1 est IL-1β.

6. Utilisation selon l'une quelconque des revendications précédentes, dans laquelle l'état est une septicémie.

7. Utilisation selon l'une quelconque des revendications 1 à 5, dans laquelle l'état est un choc septique.

8. Utilisation selon l'une quelconque des revendications précédentes, dans laquelle l'état est induit par un ligand dé récepteur apparenté à Toll (TRR).

9. Utilisation selon l'une quelconque des revendications précédentes, dans laquelle l'état est induit par un lipopolysaccharide (LPS).

10. Utilisation selon l'une quelconque des revendications précédentes, dans laquelle l'état est induit par le zymosan.

11. Utilisation selon l'une quelconque des revendications 1 à 9, dans laquelle l'état est induit par des bactéries gram-négatives.

12. Utilisation selon l'une quelconque des revendications précédentes, dans laquelle l'inhibiteur est spécifique d'un membre ou de membres de la famille Tec des PTK.

13. Utilisation selon l'une quelconque des revendications précédentes, dans laquelle le membre de la famille Tec des PTK est la tyrosine kinase de Bruton (Btk).

14. Utilisation selon l'une quelconque des revendications 1 à 12, dans laquelle le membre de la famille Tec des PTK est Tec.

15. Utilisation selon l'une quelconque des revendications précédentes, dans laquelle l'inhibiteur est un inhibiteur chimique.

16. Utilisation selon la revendication 15, dans laquelle l'inhibiteur est LFM-A13.

17. Utilisation selon l'une quelconque des revendications 1 à 14, dans laquelle l'inhibiteur est un anticorps ou l'un de ses fragments qui est capable de se lier spécifiquement à un membre ou à des membres de la famille Tec des PTK ou à l'un de leurs fragments.

18. Utilisation selon l'une quelconque des revendications 1 à 14, dans laquelle l'inhibiteur est un acide nucléique capable de réduire l'expression d'un membre ou de membres de la famille Tec des PTK.

19. Utilisation selon la revendication 18, dans laquelle l'acide nucléique comprend la séquence ou la séquence complémentaire telle que définie dans l'une quelconque des figures 11, 13, 15 ou 18, ou la séquence d'un acide nucléique qui code un polypeptide tel que défini dans l'une quelconque des figures 12, 14, 16, 17 ou 19, ou l'une de ses séquences complémentaires, un variant de l'une quelconque de ces séquences ou un fragment de l'un quelconque des éléments ci-dessus.

20. Méthode pour identifier un inhibiteur d'un membre ou de membres de la famille Tec des PTK, cet inhibiteur convenant à une utilisation dans le traitement d'un état associé à une production de cytokines, ledit état étant une polyarthrite rhumatoïde ou un état résultant d'une infection, la méthode comprenant :
(a) la fourniture, en tant que premier composant, d'une cellule capable de production de cytokines induite par un ligand de TRR ;
(b) la fourniture, en tant que second composant, d'un ligand de TRR ;
(c) la mise en contact du premier et du second composants en présence d'un agent à tester ;
(d) la détermination si l'agent à tester est capable d'inhiber l'activité induite par un ligand de TRR d'un membre de la famille Tec des PTK ; pour déterminer ainsi si l'agent à tester pourrait agir comme inhibiteur de la production de cytokines.

21. Méthode selon la revendication 20, comprenant en outre l'étape consistant à déterminer si l'inhibiteur est spécifique des PTK de la famille Tec.

22. Utilisation d'un inhibiteur d'un membre ou de membres de la famille Tec des PTK pour étudier l'inhibition d'une septicémie et/ou d'un choc septique provoqué par un ligand de TRR, *in vitro*.
